# FASCICULE DE BREVET EUROPEEN

(11) **EP 1 663 219 B1**
(45) Date de publication et mention de la délivrance du brevet: **12.05.2010**
(21) Numéro de dépôt: 04787316.1
(22) Date de dépôt: 06.09.2004
(51) Int. Cl.: A61K 31/437, A61K 31/403

(54) **DERIVES D'INDOLE POUR LE TRAITEMENT DE MALADIES LIEES AU PROCESSUS D'EPISSAGE**
VERWENDUNG VON VERBINDUNGEN AUS INDOL ZUR HERSTELLUNG EINES MEDIKAMENTS ZUR BEHANDLUNG VON ERKRANKUNGEN IM ZUSAMMENHANG MIT DEM SPLICING-VERFAHREN
USE OF INDOLE-DERIVED COMPOUNDS FOR THE PREPARATION OF A MEDICAMENT THAT CAN BE USED TO TREAT DISEASES RELATED TO THE SPLICING PROCESS

(30) Priorité: 04.09.2003 FR 0310460; 02.02.2004 FR 0400973
(43) Date de publication de la demande: 07.06.2006
(73) Titulaire: CENTRE NATIONAL DE LA RECHERCHE SCIENTIFIQUE (CNRS), 75016 Paris (FR); Universite Montpellier 2 Sciences Et Techniques, 34000 Montpellier (FR); Institut Curie, 75005 Paris (FR)
(72) Inventeur: TAZI, Jamal, F-34830 Clapiers (FR); SORET, Johann, F-34820 Teyran (FR); JEANTEUR, Philippe, F-34980 Montferrier (FR); GRIERSON, David, F-78000 Versailles (FR); RIVALLE, Christian, 75015 Paris (FR); BISAGNI, Emile, 91400 Orsay (FR); NGUYEN, Chi Hung, 92160 Antony (FR)
(74) Mandataire: Warcoin, Jacques
(86) Numéro de dépôt international: PCT/FR2004/002261
(87) Numéro de publication internationale: WO 2005/023255

(56) Documents cités:
- EP-A- 0 010 029
- EP-A- 0 394 112
- FR-A- 2 387 229
- FR-A- 2 422 662
- FR-A- 2 485 537
- FR-A- 2 627 493
- OHASHI M ET AL: "Ellipticine and related anticancer agents" EXPERT OPINION ON THERAPEUTIC PATENTS 1996 UNITED KINGDOM, vol. 6, no. 12, 1996, pages 1285-1294, XP008034013 ISSN: 1354-3776
- KANSAL V K ET AL: "THE BIOGENETIC SYNTHETIC AND BIOCHEMICAL ASPECTS OF ELLIPTICINE AN ANTITUMOR ALKALOID" TETRAHEDRON, ELSEVIER SCIENCE PUBLISHERS, AMSTERDAM, NL, vol. 42, no. 9, 1986, pages 2389-2408, XP002293633 ISSN: 0040-4020
- PINDUR U ET AL: "ANTITUMOR ACTIVE DRUGS AS INTERCALATORS OF DEOXYRIBONUCLEIC ACID MOLECULAR MODELS OF INTERCALATION COMPLEXES" JOURNAL OF CHEMICAL EDUCATION, AMERICAN CHEMICAL SOCIETY, US, vol. 70, no. 4, avril 1993 (1993-04), pages 263-272, XP008034012 ISSN: 0021-9584
- PODDEVIN B ET AL: "DUAL TOPOISOMERASE I AND II INHIBITION BY INTOPLICINE (RP-60475), A NEW ANTITUMOR AGENT IN EARLY CLINICAL TRIALS" MOLECULAR PHARMACOLOGY, BALTIMORE, MD, US, vol. 44, no. 4, 1 octobre 1993 (1993-10-01), pages 767-774, XP002071386 ISSN: 0026-895X
- TAZI J ET AL: "DNA topoisomerase I: customs officer at the border between DNA and RNA worlds?" JOURNAL OF MOLECULAR MEDICINE (BERLIN, GERMANY) 1997 NOV-DEC, vol. 75, no. 11-12, novembre 1997 (1997-11), pages 786-800, XP002293630 ISSN: 0946-2716
- HAEGG M ET AL: "INDUCTION OF ENDOPLASMIC RETICULUM STRESS BY ELLIPTICINE PLANT ALKALOIDS" MOLECULAR CANCER THERAPEUTICS, AMERICAN ASSOCIATION OF CANCER RESEARCH, US, vol. 3, no. 4, 2004, pages 489-497, XP008054026 ISSN: 1535-7163

## Description

L'invention se rapporte à de nouveaux composés dérivés d'indole et leur utilisation pour la préparation d'un médicament utile pour traiter des maladies liées au processus d'épissage.

Certains composés dérivés d'indole tels que les dérivés d'ellipticine et d'azaellipticine sont déjà connus en tant que molécules intercalantes pour corriger le disfonctionnement de l'expression génétique, au niveau de la réplication. Elles ont été plus spécifiquement décrites pour le traitement de maladies telles que le cancer, la leucémie et le SIDA (FR 2 627 493, FR 2 645 861, FR 2 436 786).

Le processus d'épissage intracellulaire consiste à éliminer les introns des ARN pré-messagers de façon à produire un ARN messagers mature exploitable par la machinerie de traduction de la cellule (Sharp, P.A. (1994). Split gènes and RNA splicing. Cell 77, 805-815). Dans le cas d'épissages alternatifs, un même précurseur peut être à l'origine d'ARN messagers codant pour des protéines ayant des fonctions distinctes (Black, D.L Mechanisms of Alternative Pre-Messenger RNA Splicing. Annu.Rev.Biochem.2003. 72,291-336). La sélection précise des sites d'épissage 5' et 3' est donc un mécanisme générateur de diversité et peut conduire à une régulation de l'expression des gènes en fonction du type de tissu ou au cours du développement d'un organisme. Parmi les facteurs impliqués dans cette sélection, on trouve une famille de protéines appelées SR (Serine Arginine Rich protéine) caractérisées par la présence d'un ou deux domaine(s) de liaison à l'ARN de type-RRM (RNA Recognation Motif) et un domaine riche en résidus arginine et sérine appelé domaine RS (Argenine Serine) (Manley, J.L. and Tacke, R. (1996). SR proteins and splicing control. Genes Dev. 10, 1569-1579). En se fixant sur de courtes séquences exoniques ou introniques du pre-mRNA, appelées ESE (Exonic Splicing Enhancer) ou ISE (Intronic Splicing Enhancer), les protéines SR sont capables d'activer, de façon dose-dépendante, des sites d'épissages suboptimaux et de permettre l'inclusion d'exons (Graveley, B.R. Sorting out the complexity of SR protein functions. RNA.2000. 6, 1197-1211). L'activité d'une protéine SR dans l'épissage alternatif est spécifique dans la mesure où l'inactivation du gène correspondant est létal (Wang, H.Y. et al., SC35 plays a role in T cell development and alternative splicing of CD45. Mol.Cell 2001. 7, 31-342).

Le séquençage du génome humain et l'analyse des banques d'EST (Expressed Sequence Tag) ont révélé que 35 à 65% des gènes s'expriment sous la forme de variants d'épissage alternatif (Ewing, B. and Green, P. Analysis of expressed sequence tags indicates 35,000 human genes. Nat.Genet.2000. 25, 232-234). Ce mécanisme est donc une cible privilégiée d'altérations qui peuvent affecter les facteurs impliqués dans la régulation de l'épissage et de mutations qui touchent les séquences nécessaires à cette régulation. A l'heure actuelle, on estime qu'environ 50 % des mutations ponctuelles responsables de maladies génétiques induisent un épissage aberrant. Ces mutations peuvent interférer avec l'épissage en inactivant ou en créant des sites d'épissage, mais aussi en modifiant ou en générant des éléments régulateurs de type «Splicing Enhancer » ou « Splicing Silencer » dans un gène particulier (Cartegni, L. et al., Listening to silence and understanding nonsense: exonic mutations that affect splicing.Nat.Rev.Genet.2002. 3, 285-298).

Les stratégies actuellement développées pour corriger ces défauts d'épissage reposent sur l'utilisation de différents types de molécules.

Le document de TAZI J et al. (DNA topoisomerase I : customs office rat the border between DNA and RNA worlds?, Journal of Molecular Medecine (Berlin, Germany) 1997 Nov-Dec, vol. 75, no.11-12) décrit que certains dérivés inhibent la DNA topoisomérase I, laquelle est une kinase spécifique qui phosphoryle les facteurs d'épissage SR.

Le document de PODDEVIN B et al. (Dual topoisomerase I and II inhibition by intoplicine (RP-60475), a new antitumor agent in early clinical trials, Molecular Pharmacology, Baltimore, MD, US, vol.44, no.4) décrit que l'intoplicine est une molécule qui inhibe à la fois la topoisomérase I et la topoisomérase II, démontrant que ce composé peut être actif contre les tumeurs.

Le document de Pilch B et al. (Specific inhibition of serine- and arginine-rich splicing factors phosphorylation, splicéosome assembly, and splicing by the antitumor drug NB-506, Cancer Research, 15 sep 2001, US, vol.61, no.18) décrit que des médicaments de type indolocarbazole inhibent la topoisomérase I et peuvent de ce fait être considérés comme des médicaments anti-cancéreux.

Une stratégie visant au développement de nouvelles molécules permettant de corriger ou d'éliminer les épissages anormaux reposent par exemple sur la surexpression de protéines qui interfèrent avec ce type d'épissage (Nissim-Rafinia, M. et al., Cellular and viral splicing factors can modify the splicing pattern of CFTR transcrits canying splicing mutations. Hum.Mol.Genet.2000. 9, 1771-1778 ; Hofmann, Y. et aL, Htra2-beta 1 stimulates an exonic splicing enhancer and can restore full-length SMN expression to survival motor neuron 2 (SMN2). Proc.Natl.Acad.Sci.U.S.A.2000. 97, 9618-9623).

Une autre stratégie repose sur l'utilisation d'oligonucléotides antisens (Sazani, P. et al., Systemically delivered antisense oligomers upregulate gene expression in mouse tissues. Nat.Biotechno1.2002. 20, 1228-1233 ; Sazani, P. and Kole, R. Modulation of alternative splicing by antisense oligonucleotides. Prog.Mol.Subcell.Biol.2003. 31, 217-239) ou de PNA (Peptidic Nucleic Acid) (Cartegni, L. et al., Correction of disease-associated exon skipping by synthetic exon-specific activators. Nat.Struct.Bio1.2003. 10, 120-125) permettant respectivement d'inhiber ou d'activer un événement d'épissage.

Une autre stratégie encore repose sur l'identification de composés qui influencent l'efficacité d'épissage du pré-mRNA d'intérêt (Andreassi, C. et al., Aclarubicin treatment restores SMN levels to cells derived from type I spinal muscular atrophy patients. Hum.Mol.Genet.2001. 10, 2841-2849).

Enfin, une stratégie basée sur l'utilisation de l'épissage en trans pour remplacer des exons mutés a été décrite (Liu, X. et al., Partial correction of endogenous DeltaF508 CFTR in human cystic fibrosis airway epithelia by spliceosome-mediated RNA trans-splicing. Nat.Biotechnol. 2002. 20, 47-52).

Un des inconvénient des stratégies développées et citées ci-avant pour corriger ou éliminer les épissages anormaux est leur coût de production. En effet, le coût de production des oligonucléotides antisens qui doivent être modifiés pour améliorer leur stabilité ou encore celui des molécules de type PNA est élevé.

Un autre inconvénient des stratégies développées et citées ci-avant est qu'elles requièrent l'utilisation de vecteurs d'expression, comme par exemple pour la stratégie basée sur l'utilisation de l'épissage en trans.

Les inventeurs se sont donnés pour but de trouver une nouvelle stratégie et notamment par l'utilisation de molécules ayant la capacité d'inhiber les processus d'épissage des ARN pré-messagers, et ne présentant pas les inconvénients des molécules de l'art antérieur.

Ainsi la présente invention concerne des dérivés d'indole correspondant à la formule I suivante : 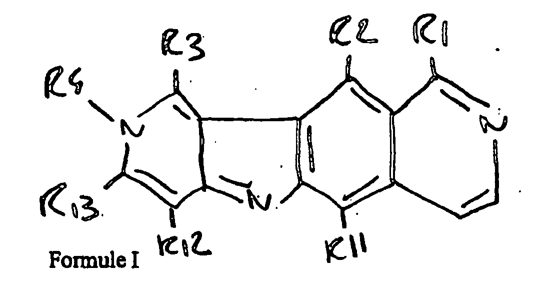 dans laquelle:
R1 représente :
- un atome d'halogène ou un groupement -C=N-OH ou -O-C(=O)(CH₃) ou -C ≡ N, ou
- un gloupement-N-R6R7,
   où R6 et R7 représentent indépendamment l'un de l'autre :
   - un atome d'hydrogène,
   - un cycle en C6, saturé ou insaturé, comportant éventuellement un atome d'azote, et éventuellement substitué par un ou plusieurs groupements alkyles en C1 à C3, ou
   - un groupement alkyle de C1 à C13 linéaire ou ramifié et/ou insaturé, dans lequel un ou plusieurs atomes de carbone peut être substitué par un atome d'azote, ledit groupement alkhyle étant éventuellement substitué par un ou plusieurs groupements -OH et/ou = O et/ou par un groupement tel que : 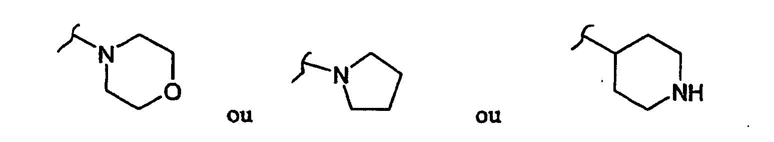
   ou 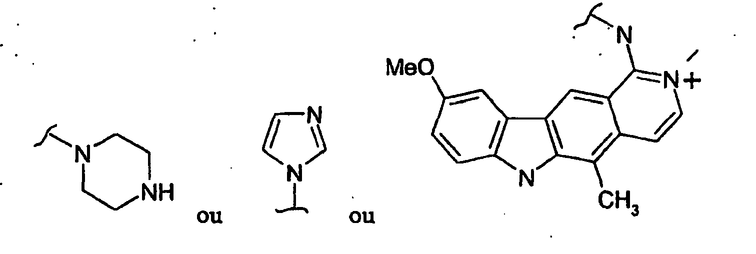 ledit groupement étant éventuellement substitué par un groupement alkyle en C1 à C3 lui-même éventuellement substitué par un groupement amine,
- un groupement-NH-R8
   où R8 représente un groupement alkyle-N-R9R10
   où le groupement alkyle représente un groupement de C1 à C13 linéaire ou ramifié éventuellement insaturé et/ou substitué par un ou plusieurs groupements alkyle en C1 à C3 et/ou groupements hydroxyle,
   R9 et R10 représentent chacun indépendamment l'un de l'autre un atome d'hydrogène ou un groupement alkyle en C1 à C4 éventuellement substitué par un ou plusieurs groupements hydroxyle et/ou oxo,
   R2 représente un atome d'hydrogène, un groupement méthyle ou un groupement - NH-(CH₂)₃-N(CH₃)₂,
   R3 représente un atome d'hydrogène, un atome d'halogène ou un groupement méthyle, amine ou méthoxyméthyle ou -NH-R8 tel que défini précédemment,
   R4 représente un groupement hydroxyle ou alkyle en C1-C6 ou un groupement méthoxy éventuellement substitué par un groupement phényle,
   R11 et R12 représentent indépendamment l'un de l'autre un atome d'hydrogène ou un groupement alkyle en C1-C3,
   R13 représente un atome d'hydrogène ou un groupement méthyle, et les sels pharmaceutiquement acceptables desdits composés, et/ou mélanges de ceux-ci.

Par "maladies liées à l'épissage des ARN pré-messagers dans la cellule", on entend toutes les maladies liées au processus d'épis sage, c'est-à-dire les maladies dont la cause est une altération du processus d'épissage et les maladies dont l'apparition nécessite que le processus d'épissage des cellules soit activé. Notamment, on entend les maladies génétiques résultant de l'altération des processus d'épissage comme par exemple le syndrome de frasier, la démence fronto-temporale liée au chromosome 17 (une forme du Parkinson), le syndrome de Leigh (type d'encéphalopathie), la mucoviscidose atypique, certaines neuropathologies dont notamment l'Alzheimer lié à une mutation de la protéine Tau, l'amyotrophie qui touche le gène SMN (Survival of Motor Neuron), la dépression liée à un dérèglement de l'épissage de la sérotonine, et certains cancers dans lesquelles le processus global de l'épissage est affecté (notamment le cancer du sein, du colon et certains lymphomes).

On entend également les maladies d'origine virale ou dues à l'intrusion de virus dans l'organisme humain ou animal, appelées maladies virales, et pour lesquelles des séquences ESE sont identifiées pour l'épissage. En particulier, on peut citer le SIDA pour lequel des séquences ESE sont identifiées dans l'épissage de certains ARN pré-messagers de gènes clefs impliqués dans la réplication du virus responsable du SIDA.

Par "atome d'halogène", on entend le groupe F, Cl, Br et I, et plus particulièrement le Cl.

Par "anhydro base", on entend un composé résultant de la neutralisation acido-basique interne (avec perte d'eau) des hydroxydes iminium contenant un site acide conjugué avec la fonction iminium (cf. IUPAC). Dans le cas de la présente invention, dans l'anhydro base, l'hydroxyde est remplacé par l'amino.

Le premier avantage lié à l'utilisation de dérivés d'indole tels que dérivés de benzo-indole ou de pyrido-indole selon l'invention pour traiter des maladies liées au processus d'épissage est d'ordre financier. En effet, le coût de production de ces molécules est bien inférieur à celui des oligonucléotides antisens ou encore à celui des molécules hybrides de type PNA.

Le second avantage des dérivés d'indole selon l'invention tient à leur facilité d'administration et au fait que cette stratégie de traitement ne requiert pas l'utilisation de vecteurs d'expression.

La pénétration des molécules selon l'invention à l'intérieur des cellules et leur ciblage vers des tissus particuliers peuvent être effectués soit en utilisant des polymères (Uekama, K. et al., Cyclodextrins in drug carrier systems. Crit.Rev.Ther.Drug Carrier.Syst. 1987. 3, 1-40), soit des vecteurs tels que peptides ou lipides (Prochientz, A. Getting hydrophilic compounds into cells: lessons from homeopeptides. Curr.Opin.Neurobiol. 1996. 6, 629-634 et Vives, E. et al., A truncated HIV-1 Tat protein basic domain rapidly translocates through the plasma membrane and accumulates in the cell nucleus. J.Biol.Chem. 1997. 272, 16010-16017) ou soit encore des particules telles que les nanoparticules et les lipsomes (Douglas, S.J. et al., Nanoparticles in drug delivery. Crit.Rev.Ther.Drug Carrier.Syst. 1987. 3, 233-261 et Gregoriadis, G. et al., Liposomes in drug delivery. Clinical, diagnostic and ophthalmic potential. Drugs 1993. 45, 15-28).

Dans un autre mode de réalisation préférentielle, dans la formule I,
R1 représente un atome d'halogène, un groupement amine, -N-R₆R₇ ou -NH-R8,
R2 représente un atome d'hydrogène ou un groupement méthyle,
R3 représente un atome d'hydrogène ou un groupement NH-R8,
R4 représente un groupement méthyle,
R11 représente un atome d'hydrogène ou un groupement méthyle, et
R6, R7, R8, R9, R10, R12 et R13 sont tels que définis précédemment.

Le composé préférentiel est:
- la 10-chloro-2,6-diméthyl-2H-pyrido[3',4':4,5]pyrrolo[2,3-g]isoquinoline,
- la N,N-diéthyl-N'-(6-méthyl-5H-pyrido[3',4':4,5]pyrrolo[2,3-g]isoquinolin-10-yl)-éthane-1,2-diamine,
- la N'-(5,6-diméthyl-5H-pyrido[3'4':4,5]pyoolo[2,3-g]isoquinolin-10-yl)-N,N-diméthyl-propane-1,3-diamine,

Un autre objet de l'invention est les composés tels que décrits précédemment en tant que médicament.

Les composés selon l'invention ont la capacité d'inhiber les processus d'épissage des ARN pré-messagers qui sont soit constitutifs, soit, de manière plus spécifique, dépendants de séquences régulatrices appelées ESE (Exonic Splicing Enhancer), ISE (Intronic Splicing Enhancer), ESS (Exonic Splicing Silencer) et ISS (Intronic Splicing Silencer).

Les processus d'épissage sont soit constitutifs et/ou soit dépendants de séquences régulatrices ESE. Un autre objet de l'invention est les composés tels que décrits précédemment pour traiter les maladies liées au processus d'épissage des ARN pré-messagers dans la cellule, notamment les maladies génétiques résultant de l'altération des processus d'épissage, notamment le syndrome de frasier, la démence fronto-temporale liée au chromosome 17 (une forme du Parkinson), le syndrome de Leigh (type d'encéphalopathie), la mucoviscidose atypique, certaines neuropathologies dont notamment l'Alzheimer lié à une mutation de la protéine Tau, l'amyotrophie qui touche le gène SMN (Survival of Motor Neuron), la dépression liée à un dérèglement de l'épissage de la sérotonine, les maladies d'origine virale et pour lesquelles des séquences ESE sont identifiées pour l'épissage.

De manière préférentielle, la maladie virale est le SIDA.

Dans un mode de réalisation selon l'invention, ledit médicament comprend également un excipient permettant de formuler les composés selon la formule I et ledit médicament se présente sous forme solide ou liquide pour être préparé et administré par voie intraveineuse.

Les composés selon l'invention seront administrés de préférence par voie intraveineuse à une concentration de 80-100 mg/m² (cf. Paoletti C. et al., Antitumor activity, pharmacology, and toxicity of ellipticine, ellipticinium, and 9-hydroxy derivatives : preliminary clinical trials of 2-methyl-9-hydroxy ellipticinium (NSC 264-137) in recent results in Cancer Research, vol 74, pp108-123, 1980, G. Mathé and F.M. Muggia, Eds (Springer-Verlag Pbl). La concentration sera choisie par l'homme du métier selon l'organe ou tissu à traiter, l'état d'avancement de la maladie, et le mode de ciblage utilisé.

### Description des figures :

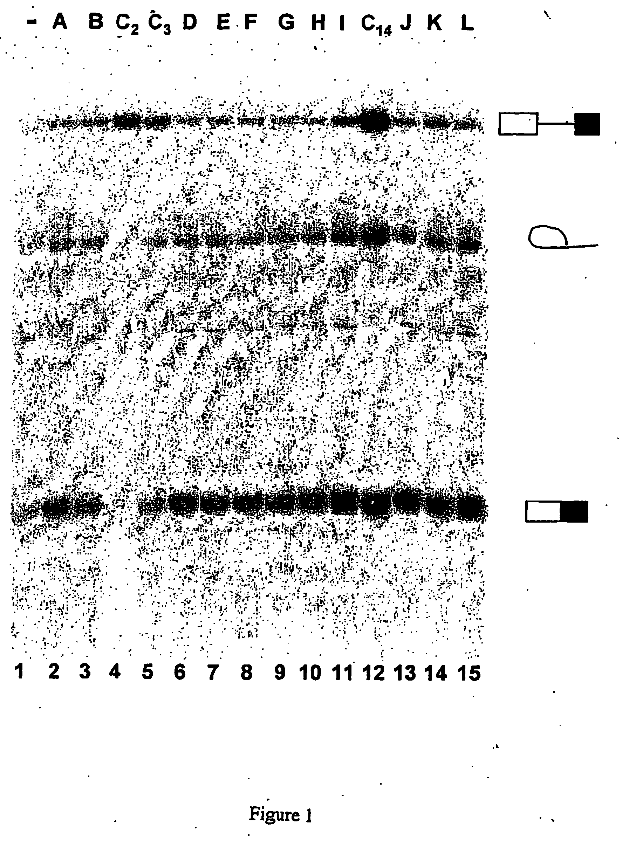

Figure 1 : Analyse des produits d'épissage de l'ARN pré-messager Minx obtenus in vitro en présence de différents composés. La structure des différents produits d'épissage est indiquée. Le trait représente l'intron soit sous forme linéaire ou en lasso (*). Les rectangles représentent les deux exons du Minx.

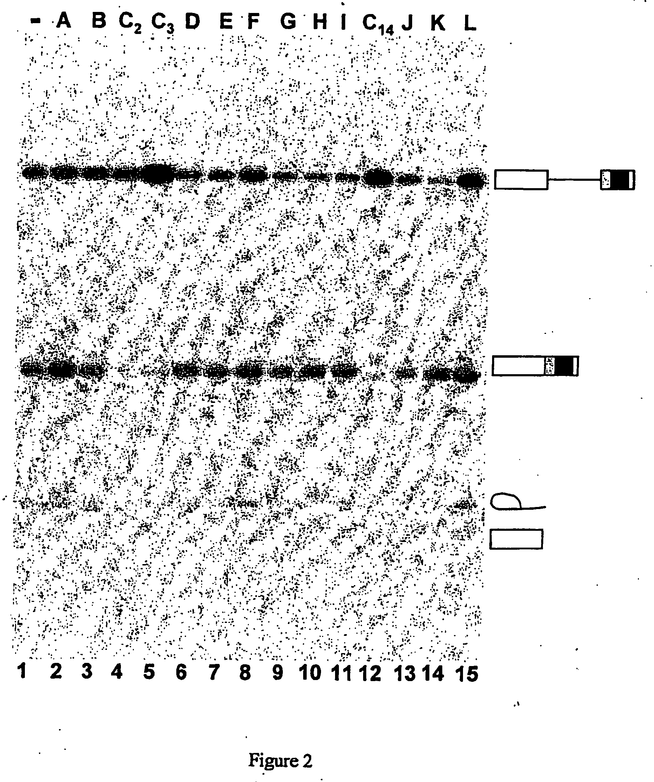

Figure 2 : Analyse des produits d'épissage de l'ARN pré-messager M3S1 obtenus in vitro en présence de différentes composés. La structure des différents produits d'épissage est indiquée. Les rectangles sont les exons. La partie noire du rectangle représente l'ESE. Le trait représente l'intron soit sous forme linéaire ou en lasso (*).

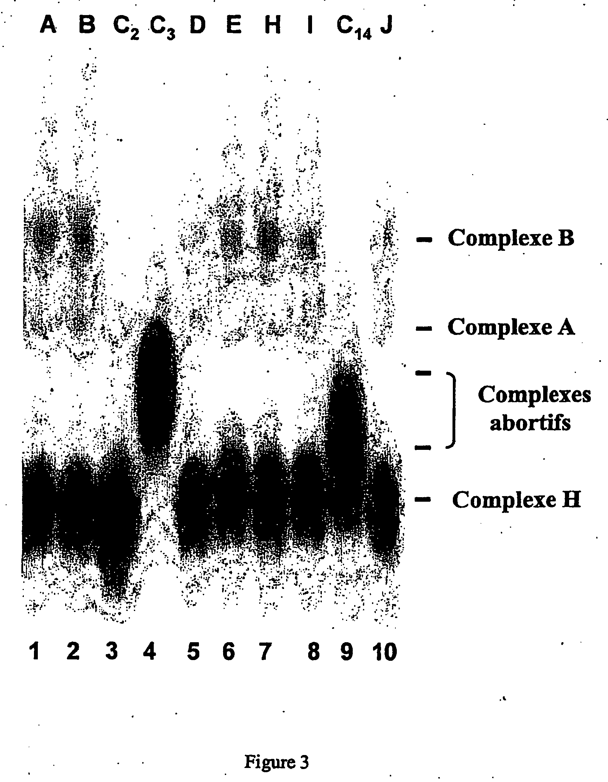

Figure 3 : Analyse de la formation des complexes d'épissage sur l'ARN pré-messager M3S1 en présence de différents composés.
Figure 4 :
   (A) Structure de deux types de transcrits produits par épissage de l'exon 7-intron 7-exon 8 du gène codant pour la sous-unité E1α de la pyruvate déshydrogénase mutée.
   (B) Analyse des produits d'épissage de l'ARN pré-messager M3S1-PDH obtenus in vitro en présence de différents composés. La structure des différents produits d'épissage est indiquée. Le trait représente les introns les rectangles représentent les trois exons.
Figure 5 :
   (A) Structure du transgène et les deux types de transcrits produits par épissage alternatif. Les flèches indiquent la position des amorces utilisées pour la PCR.
   (B) Analyse en gel d'agarose 2% des produits de PCR. M indique les marqueurs ADN correspondant à des multiples de 100 pairs de bases (pistes 1 et 6). Les PCR sont effectuées sur des ARNs issues de cellules non traitées (pistes 2 et 3), traitées par 1 µM du composé C₂₇ (piste 4) ou par 1 µM du composé C₁₄ (piste 5).

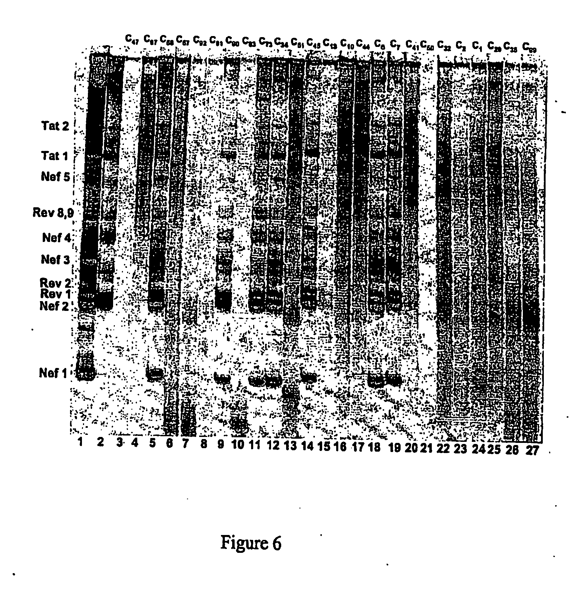

Figure 6 : Analyse des produits d'amplification par RT-PCR des mRNAs viraux issues de cellules U1 stimulées par le PMA (pistes 1, 3-27) en absence (piste 1) ou en présence (pistes 3-27) des différents composés. La piste 2 représente les produits d'amplification à partir de cellules non stimulées par le PMA. La nomenclature de RT-PCR est en accord avec Purcell D.F. and Martin M.A. (1993, Alternative splicing of human immunodeficiency virus type 1 mRNA modulates viral protein expression, replication, and infectivity. J Virol. 67:6365-78).

### Exemple 1 : Inhibition in vitro de l'épissage de deux types de pré-ARNm modèles

Les composés présentés dans les Tableaux 1 et 2 ci-après ont été testés dans des gammes de concentration de 1 µM, 10 µM et 100 µM, et sont sélectionnés dans un premier temps sur la base de leur capacité d'inhiber, in vitro, l'épissage de deux types de pré-ARNm modèles.

**Tableau 1 :**

| CODE des MOLECULES | FORMULE CHIMIQUE | NOMENCLATURE |
|---|---|---|
| **C3** | 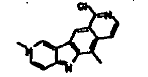 | 10-chloro-2,6-diméthyl-2H-pyride [3',4':4,5]pyrrolo[2,3-g]isoquineline |

**Tableau 2 : Composés de référence**

| CODE des MOLECULES | Composés de référence FORMULE CHIMIQUE | NOMENCLATURE |
|---|---|---|
| **C1** | 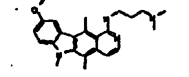 | N'-(9-méthoxy-5,6,11-triméthyl-6H-pyrido[4,3-b]carbazol-1-yl)-N,N-diméthyl-propane-1,3-diamine |
| **C2** | 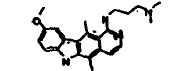 | N'-(9-méthoxy-6,11-diméthyl-6H-pyrido [4,3-b]carbazol-1-yl)-N,N-diméthyl-propane-1,3-diamine |
| **C4** | 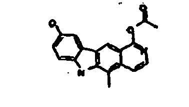 | ester 9-hydroxy-5-méthyl-6H-pyrido [4,3-b]carbazol-1-yl de l'acide acétique |
| **C5** | 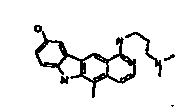 | 1-(3-diméthylamio-proplyamino) 5-méthyl-6H-pyido[4,3-b]carbazol-9-ol |
| **C6** | 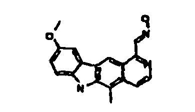 | 9-méthoxy-5-méthyl-6H-pyrido[4,3-b] carbazole-1-carbaldéyde oxime |
| **C7** | 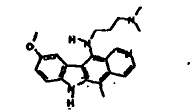 | N'(9-méthoxy-5-méthyl-6H-pyrido-[4,3-b] carbazol-11-yl)-N,N-diméthyl-propane-1,3-diamine |
| **C8** | 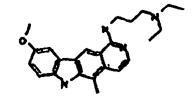 | N,N-diéthyl-N'-(9-méthoxy-5-méthyl-6H-pyrido[4,3-b]carbazol-1-yl)-propane-1,3-diamine |
| **C9** | 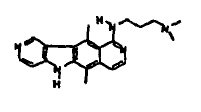 | N'-(6,11-diméthyl-5H-pyridop[3',4':4,5] pyrrolo[2,3-g]isoquinolin-10-yl)-N,N-diméthyl-propane-1,3-diamine |
| **C10** | 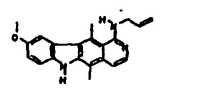 | allyl-(9-méthoxy-5,11-diméthyl-6H-pyrido[4,3-b]carbazol-1-yl)-amine |
| **C11** | 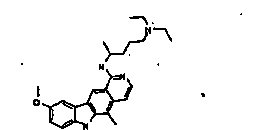 | N,N-Diéthyl-N4-(9-méthoxy-5-méthyl-6H-pyrido[4,3-b]carbazol-1-yl)-pentane-1,4-diamine |
| **C12** | 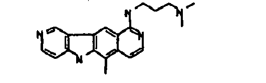 | N,N-diméthyl-N'-(6-méthyl-5H-pyrido [3',4':4,5]pyrrolo[2,3-g]isoquinolin-10 -yl)-propane-1,3-diemine |
| **C13** | 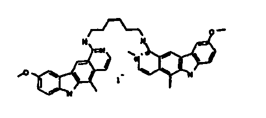 | iodure 9-méthoxy-1-[6-(9-méthoxy-5-méthyl-6H-pyrido[4,3-b]carbazol-1-ylamino)-hexylamino]-2,5-diméthyl-6H-pyrido[4,3-b]carbazol-2-ium |
| **C14** | 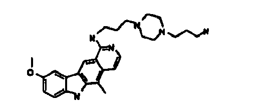 | {3-[4(3-amino-propyl)-pipérazin-1-yl]-propyl}-9-méthoxy-5-méthyl-6H-pyrido[4,3-b]carbazol-1-yl)-amine |
| **C15** | 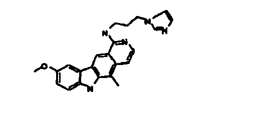 | (3-imidazol-1-yl-propyl)-9 -méthoxy-5-méthyl-6H-pyrido [4,3-b]carbanzol-1-yl)-amine |
| **C16** | 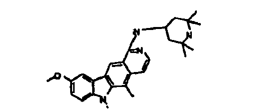 | (9-méthoxy-5-méthyl-6H-pyrido [4,3-b]carbazol-1-yl-(2,2,6,6-tétraméthyl-pipéridin-4-yl)amine |
| **C17** | 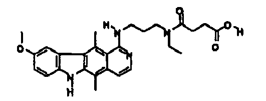 | acide N-éthyl-N-[3-(9-méhoxy-5,11-diméthyl-6H-pyrido[4,3-b]carbazol-1-ylamino)-propyl]-succinamique |
| **C18** | 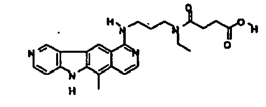 | acide N-éthyl-N-[3-(6-méthyl-5H-pyrido [3',4':4,5]pyrrolo[2,3-g]isoquinolin-10-ylamino)-propyl]-succinamique |
| **C19** | 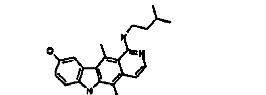 | 5,11-diméthyl-1-(3-méthyl-butylamino)-6H-pyrido[4,3-b]carbazol-9-ol |
| **C20** | 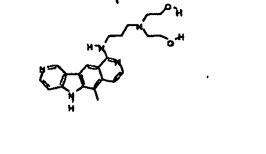 | 2-{(2-hydroxy-éthyl)-[3-(6-méthyl-5H-pyrido[3',4':4,5]pyrrolo[2,3-g]isoquinolin-10-ylamino}propyl]-amino-éthanol |
| **C21** | 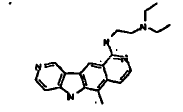 | N,N-diéthyl-N'-(6-méthyl-5H-pyrido [3',4':4,5]pyrrolo[2,3-g]isoquinolin-10-yl)-éthane-1,2-diamine |
| **C22** | 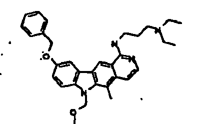 | N'-(9-benzyloxy-6-méthoxyméthyl-5-méthyl-6H-pyrido[4,3-b]carbazol-1-yl)-N,N-diéthyl-propane-1,3-diamine |
| **C23** | 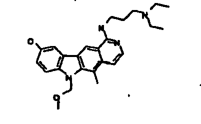 | 1-(3-diéthylamino-propylamino)-6-méthoxyméthyl-5-méthyl-6H-pyrido[4,3-b]carbazol-9-ol |
| **C24** | 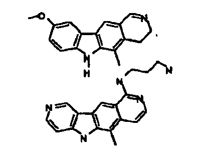 | 9-méthoxy-5-méthyl-4,6-dihydro-3H-pyrido[4,3-b]carbazole |
| **C25** | | N-(6-méthyl-5H-pyrido[3',4':4,5] pyrrolo[2,3-g]isoquinolin-10-yl) -propane-1,3-diamine |
| C26 | 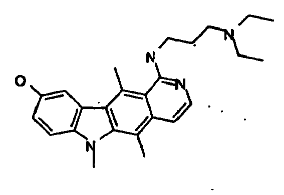 | 1-(3-diéthylamino-propylamino)-5,11-diméthyl-6H-pyrido[4,3-b] carbazol-9-ol |
| C27 | 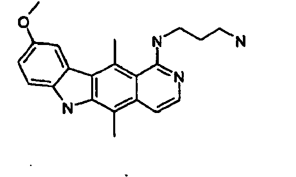 | N-(9-méthoxy-5,11-diméthyl-6H-pyrido[4,3-b]carbazol-1-yl)-propane-1,3-diamine |
| C28 | 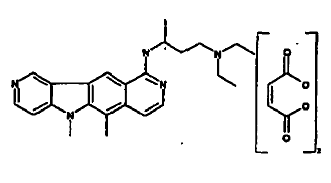 | N-3-(5,6-diméthyl-5H-pyrido[3',4':4,5] pyrrolo[2,3-g]isoquinolin-10-yl)-N, N-1-diéthyl-butane-1,3-diamine |
| C29 | 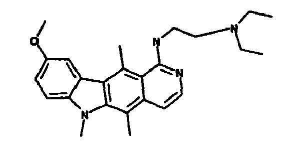 | N,N-diéthyl-N'-(9-méthoxy-5,6,11-triméthyl-6H-pyrido[4,3-b]carbazol-1-yl)-éthane-1,2-diamine |
| C30 | 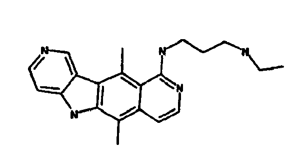 | N-(6,11-diméthyl-5H-pyrido[3',4':4,5] pyrrolo[2,3-g]isoquinolin-10-yl)-N'-éthyl-propane-1,3-diamine |
| C31 | 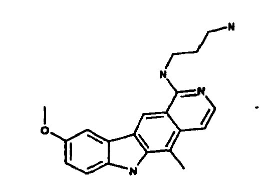 | N-(9-méthoxy-5-méthyl-6H-pyrido [4,3-b]carbazol-1-yl)-propane-1,3-diamine |
| C32 | 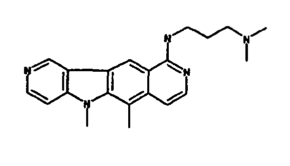 | N'-(5,6-diméthyl-5H-pyrido[3'4':4,5] pyrrolo[2,3-g]isoquinolin-10-yl)-N,N-diméthyl-propane-1,3-diemine |
| C33 | 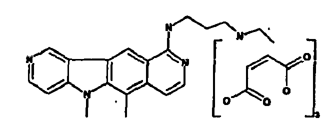 | N-(5,6-diméthyl-5H-pyrido[3'4':4,5] pyrrolo[2,3-g]isoquinolin-10-yl)-N'-éthyl-propane-1,3-diamine |
| C34 | 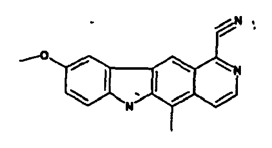 | 9-méthoxy-5-méthyl-6H-pyrido [4,3-b]carbazole-1-carbonitrile |
| C35 | 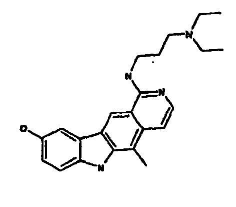 | 1-(3-diéthylamino-propylamino)-5-méthyl-6H-pyrido[4,3-b]carbazol-9-ol |
| C36 | 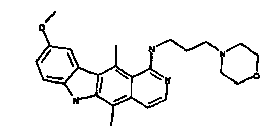 | (9-méthoxy-5,11-diméthyl-6H-pyrido [4,3-b]carbazol-1-yl)-(3-morpholin-4-yl-propyl)-amine |
| C37 | 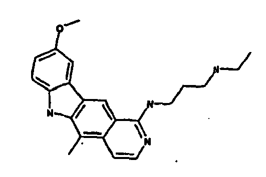 | N-éthyl-N'-(9-méthoxy-5-méthyl-6H-pyrido[4,3-b]carbazol-1-yl)-propane-1,3-diamine |
| C38 | 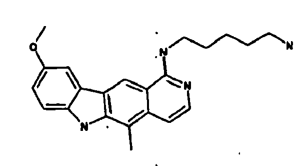 | N-(9-méthoxy-5-méthyl-6H-pyrido[4,3-b]carbazol-1-yl)-pentane-1,5-diamine |
| C39 | 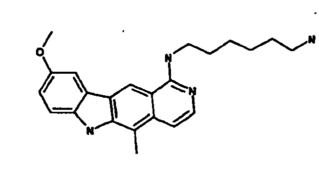 | N-(9-méthoxy-5-méthyl-6H-pyrido[4,3-b]carbazol-1-yl)-hexane-1,6-diamine |
| C40 | 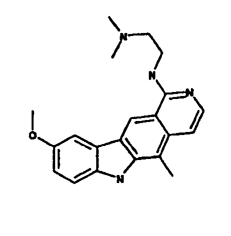 | N'-(9-méthoxy-5-méthyl-6H-pyrido [4,3-b]carbazol-1-yl)-N,N-diméthyl- éthane-1,2-diamine |
| C41 | 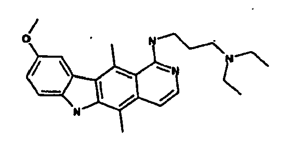 | N,N-diéthyl-N'-(9-méthoxy-5,11- diméthyl-6H-pyrido[4,3-b] carbazol-1-yl)-propane-1,3-diamine |
| C42 | 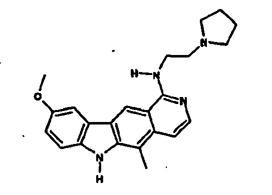 | (9-méthoxy-5-méthyl-6H-pyrido [4,3-b]carbazol-1-yl)-(2-pyrrolidin- 1-yl-éthyl)-amine |
| C43 | 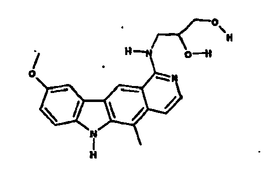 | 3-(9-méthoxy-5-méthyl-6H-pyrido [4,3,b]carbazol-1-ylamino)-propane- 1,2-diol |
| C44 | 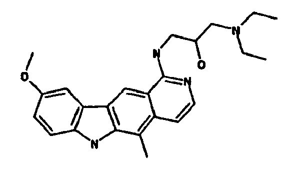 | 1-diéthylamino-3-(9-méthoxy-5- méthyl-6H-pyrido[4,3-b]carbazol- 1-ylamino)-propan-2-ol |
| C45 | 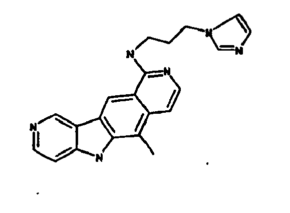 | (3-imidazol-1-yl-propyl)-(6-méthyl- 5H-pyrido[3',4':4,5]pyrrolo[2,3-g] isoquinolin-10-yl)-amine |
| C46 | 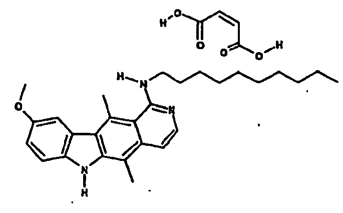 | décyl-(9-méthoxy-5,11-diméthyl- 6H-pyrido[4,3-b]carbazol-1-yl)-amine |
| C47 | 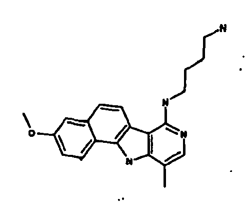 | N-(3-méthoxy-10-méthyl-11H- benzo[g]pyrido[4;3-b]indol-7-yl)- butane-1,4-diamine |
| C48 | 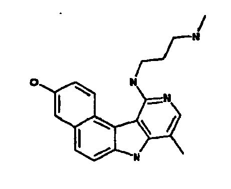 | 8-méthyl-11-(3-méthylamino- propylamino)- 7H-benzo[e]pyrido[4,3-b]indol-3-ol |
| C49 | 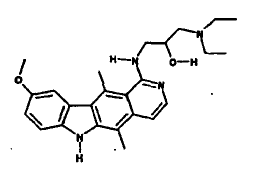 | 1-diéthylamino-3-(9-méthoxy-5,11- diméthyl-6H-pyrido[4,3-b]carbazol- l-ylamino)-propan-2-ol |
| C50 | 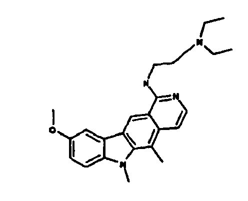 | N,N-diéthyl-N'-(9-méthoxy-5,6- diméthyl-6H-pyrido[4,3-b]carbazol- 1-yl)-éthane-1,2-diamine |
| C51 | 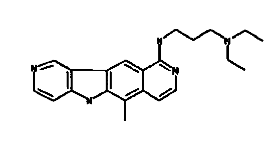 | N,N-diéthyl-N'-(6-méthyl-5H-pyrido [3',4':4,5]pyrrrolo[2,3-g]isoquinoline- 10-yl)-propane-1,3-diamine |
| C52 | 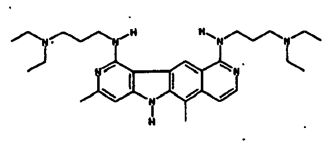 | N-1,N-10-Bis-(3-diéthylamino-propyl)- 3,6-diméthyl-5H-pyrido[3',4':4,5] pyrrolo[2,3-g]isoquinoline-1,10- diamine |
| C53 | 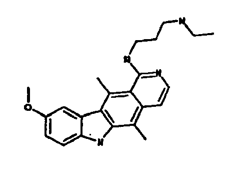 | N-éthyl-N'-(9-méthoxy-5,11- diméthyl-6H-pyrido[4,3-b]carbazol- 1-yl)-propane-1,3-diamine |
| C54 | 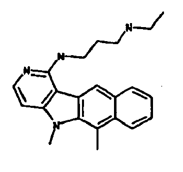 | N-(5,6-diméthyl-5H-benzo [f]pyrido[4,3]indol-1-yl)-N'-éthyl- propane-1,3-diemine |
| C55 | 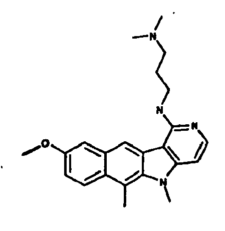 | N'-(9-méthoxy-5,6-diméthyl-5H-benzo [f]pyrido[4,3-b]indol-1-yl)-N,N-diméthyl- propane-1,3-diamine |
| C56 | 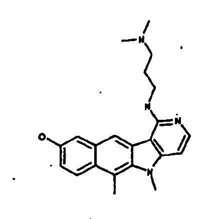 | 1-(3-diméthylamino-propylamino)- 5,6-diméthyl-5H-benzo[f]pyrido [4,3-b]indol-9-ol |
| C57 | 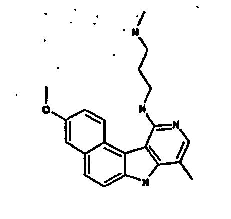 | N-(3-méthoxy-8-méthyl-7H-benzo [e]pyrido[4,3-b]indol-11-yl)-N'- méthyl-propane-1,3-diamine |
| C58 | 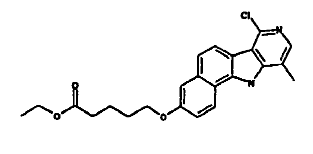 | ester éthylique de l'acide 5-(7-chloro- 10-méthyl-11H-benzo[g]pyrido[4,3-b] indol-3-yloxy)-pentanoïque |
| C59 | 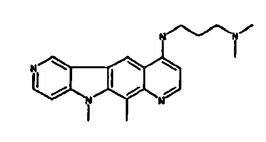 | N,N-dimétbyl-N'-(10,11-diméthyl- 10H-pyrido[3'4':4,5]pyrrolo[3,2-g] quinoline)4-yl-propane-1,3-diamine |
| C60 | 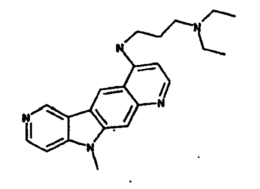 | N,N-diéthyl-N'-(10,11-diméthyl-10H- pyndo[3'4':4,5]pyrrolo[3,2-]quinoline)- 4-yl-propane-1,3-diamine |
| C61 | 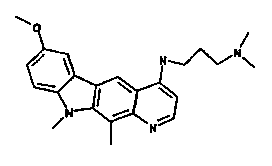 | N'-(7-méthoxy-10,11-diméthyl- 10H-pyrido[23-b]carbazol-4-yl)-N,N- diméthyl-propane-1,3-diamine |
| C62 | 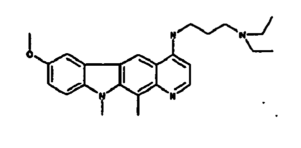 | N,N-diéthyl-N'-(7-méthoxy-10,11- iméthyl-10H-pyrido[2,3-b]carbazol-4-yl) propane-1,3-diamine |
| C63 | 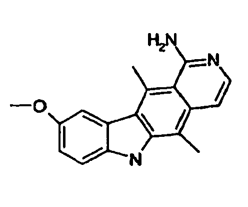 | 9-méthoxy-5,11-diméthyl-6H-pyrido [4,3-b]carbazol-1-yl-amine |
| C64 | 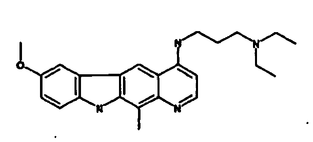 | N,N-diéthyl-N'-(7-méthoxy-11-méthyl- 10H-pyrido[2,3-b]carbazol-4-yl)- propane-1,3-diamine |
| C65 | 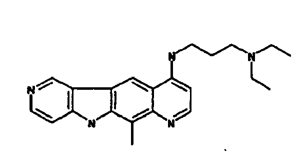 | N,N-diéthyl-N'-(11-méthyl-10H- pyrido[3',4':4,5]pyrrolo[3,2-g]quinoline)- 4-yl-propane-1,3-diamine |
| C66 | 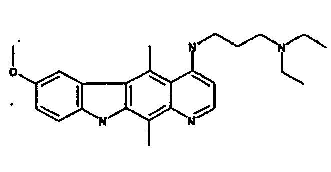 | N,N-diéthyl-N-(7-méthoxy-5,11- diméthyl-10H-pyrido[2,3-b]carbazol- 4yl)-propane-1,3-diamine |
| C67 | 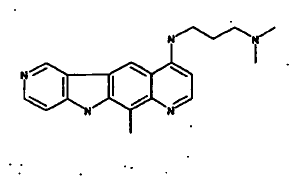 | N,N-diméthyl-N'-(11-méthyl-10H- pyrido [3',4':4,5]pyrrolo[3,2-g]quinoline)-4-yl- propane-1,3-diamine |
| C68 | 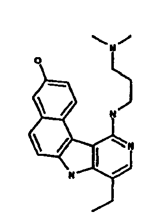 | 11-(3-diméthylemino-propylamino)- 8-éthyl-7H-benzo[e]pyrido[4,3-b] indol-3-ol |
| C69 | 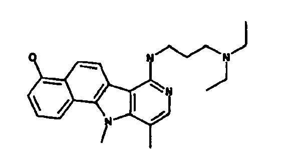 | 7-(3-diéthylamino-propylamino)10,11- diméthyl-11H-benzo[g]pyrido[4,3-b] indol-4-ol |
| C70 | 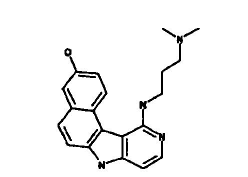 | 11-(3-diméthylamino-propylamine)- 7H-benzo[e]pyrido[4,3-b]indol-3-ol |
| C71 | 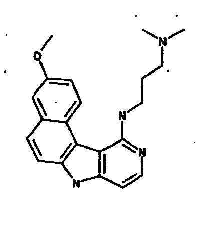 | N'-(3-méthoxy-7H-benzo[e]pyrido [4,3-b]indol-11-yl)-N,N-diméthyl- Propane-1,3-diamine |
| C72 | 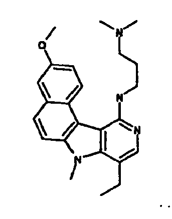 | N'-8-éthyl-3-méthoxy-7-méthyl-7H- benzo[e]pyrido[4,3-b]indol-11-yl)- N,N-diméthyl-propane-1,3-diemine |
| C73 | 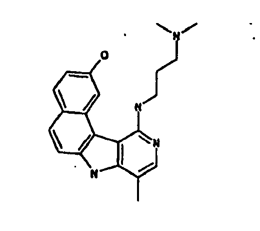 | 11-(3-diméthylamino-propylamino)- 8-méthyl-7H-benzo[e]pyrido[4,3-b] indol-2-ol |
| C74 | 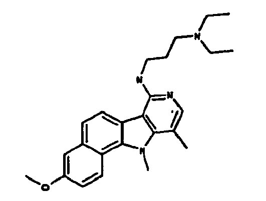 | N,N-diéthyl-N'-(3-méthoxy-10,11- diméthyl-11H-benzo[g]pyrido[4,3-b] indol-7-yl)-propane-1,3-diamine |
| C75 | 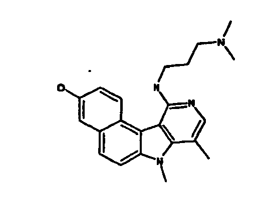 | 11-(3-diméthylamino-propylamino)- 7,8-diméthyl-7H-benzo[e]pyrido [4,3-b]indol-3-ol |
| C76 | 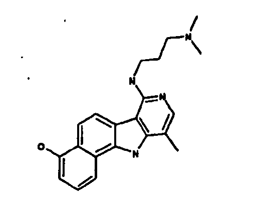 | 7-(3-diméthylamino-propylamino)- 10-méthyl-11H-benzo[g]pyido[4,3-b] indol-4-ol |
| C77 | 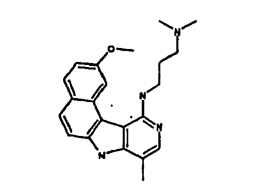 | N'-(2-méthoxy-8-méthyl-7H-benzo [e]pyrido[4,3-b]indol-11-yl)-N,N- diméthyl-propane-1,3-diamine |
| C78 | 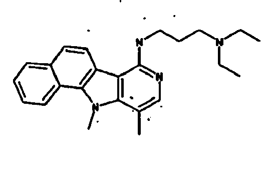 | N'-(2-méthoxy-8-méthyl-7H-benzo [e]pyrido[4,3-b]indol-11-yl)-N,N- diméthyl-propane-1,3-diamine |
| C79 | 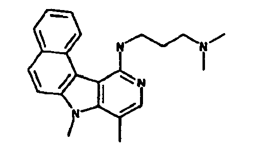 | N'-(7,8-diméthyl-7H-benzo[e]pyrido [4,3-b]indol-11-yl)-N,N-diméthyl- propane-1,3-diamine |
| C80 | 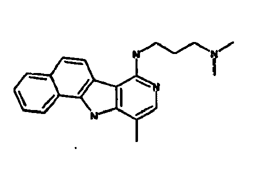 | N,N-diméthyl-N'-(10-méthyl-11H- benzo[g]pyrido[4,3-b]indol-7-yl)- propane-1,3-diamine |
| C81 | 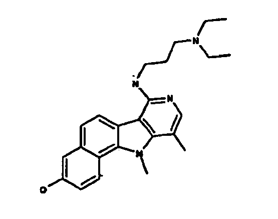 | 7-(3-diéthylamino-propylamino)- 10,11-diméthyl-11H-benzo[g]pyrido [4,3-b]indol-3-ol |
| C82 | 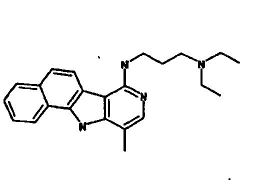 | N,N-diéthyl-N'-(10-méthyl-11H- benzo[g]pyrido[4,3-b]indo)-7-yl)- propane-1,3-diamine |
| C83 | 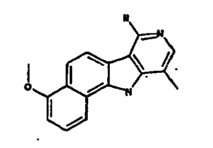 | 4-méthoxy-10-méthyl-11H-benzo [g]pyrido[4,3-b]indol-7-ylamine |
| C84 | 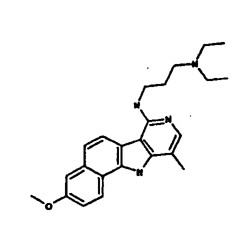 | N,N-diéthyl-N'-(3-méthoxy-10-méthyl- 11H-benzo[g]pyrodo[4,3-b]indol-7-yl)- pxpane-1,3-diumine |
| C85 | 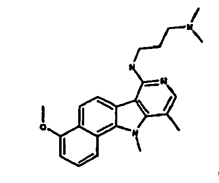 | N-(4-méthoxy-10,11-diméthyl-11H-benzo [g]pyrido[4,3-b]indol-7-yl)-N,N-diméthyl- propane-1,3-diamine |
| C86 | 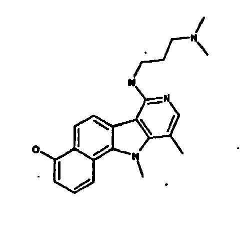 | 7-(3-(diméthylamino-propylamino)- 10,11-diméthyl-11H-benzo[g]pyrido [4,3-b]indol-4-ol |
| C87 | 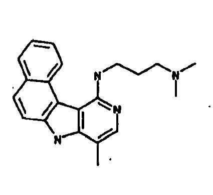 | N,N-diméthyl-N'-(8-méthyl-7H-benzo [e]pyrido[4,3-b]indol-11-yl)-propane- 1,3-diamine |
| C88 | 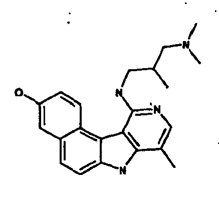 | 11-(3-diméthylamino-2-méthyl-propylamino)- 8-méthyl-7H-benzo[e]pyrido [4,3-b]indol-3-ol |
| C89 | 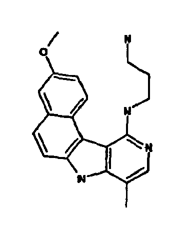 | N-(3-méthoxy-8-méthyl-7H-benzo [e]pyrido[4,3-b]indol-11-yl)-propane- 1,3-diamine |
| C90 | 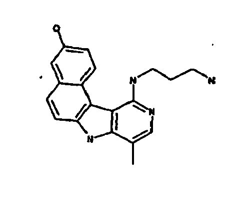 | 11-(3-amino-propylamino)-8-méthyl- 7H-benzo[e]pyrido[4,3-b]indol-3-ol |
| C91 | 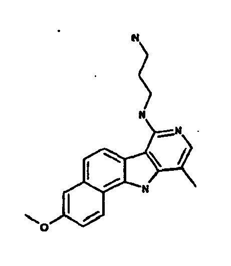 | N-(3-méthoxy-10-méthyl-11H-benzo [g]pyrido[4,3-b]indol-7-yl)-propane- 1,3-diamine |
| C92 | 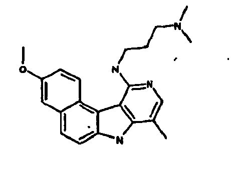 | N'-(3-méthoxy-8-méthyl-7H-benzo [e]pyrido[4,3-b]indol-11-yl)-N,N- diméthyl-propane-1,3-diamine |
| C93 | 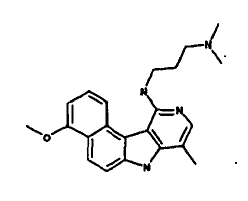 | N'-(4-méthoxy-8-méthyl-7H-benzo [e]pyrido[4,3-b]indol-11-yl)-N,N- diméthyl-propane-1,3-diamine |
| C94 | 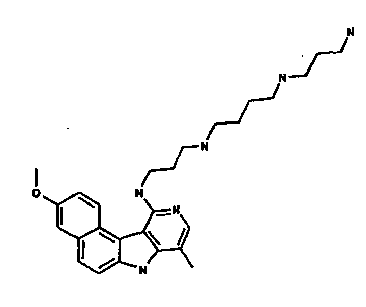 | N-(3-amino-propyl)N'-[3-(3-méthoxy-8- méthyl-7H-benzo[e]pyrido[4,3-b]indol-11- ylamino)-propyl]butane-1,4-diamine |
| C95 | 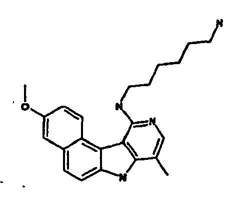 | N-(3-méthoxy-8-méthyl-7H-benzo [e]pyrido[4,3-b]indol-11-yl)-hexane- 1,6-diamine |
| C96 | 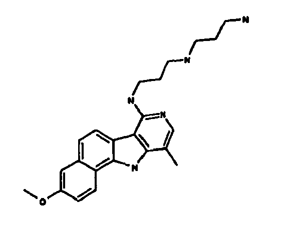 | N-[3-(3-méthoxy-10-méthyl-11H-benzo [g]pyrido[4,3-b]indol-7-ylamino)-propyl]- propane-1,3-diamine |
| C97 | 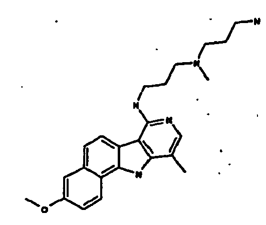 | N-[3-(3-méthoxy-10-méthyl-11H-benzo [g]pyrido[4,3-b]indol-7-ylamino)-propyl]- N-méthyl-propane-1,3-diamine |
| C98 | 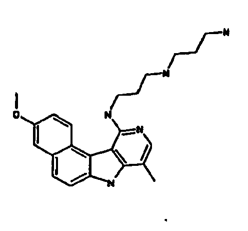 | N-[3-(3-méthoxy-8-méthyl-7H-benzo [e]pyrido[4,3-b]indol-11-ylamino)- propyl]-propane-1,3-diamine |
| C99 | 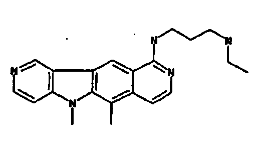 | N-(5,6-diméthyl-5H-pyrido[3',4':4,5] pyrrolo[2,3-g]isoquinolin-10-yl)-N'- éthyl-pmpane-1,3-diamine |
| C100 | 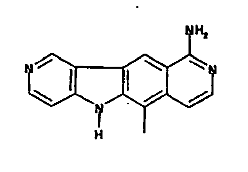 | 6-méthyl-5H-pyrido[3',4':4,5]pyrrolo [2,3-g]isoquinolin-10-yl-amine |
| A | 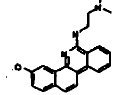 | 6-(2-Dimethylamino-ethylamino)-benz o(c)phenantridin-3-ol |
| B | 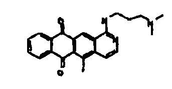 | 1-(3-Dimethylamino-propylamino)-5-m 11-dione |
| D | 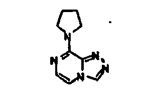 | 8-Pyrroiidin-1-yl-[1,2,4]triazolo[4 ,3-e]pyrazine |
| E | 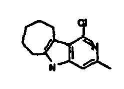 | 4-Chloro-2-methyl-5,6,7,8,9,10-here hydro-3,10-dlazo-benzo[e]azulene |
| F | 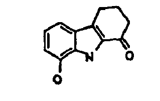 | 8-Hydroxy-2,3,4,9-tetrahydro-carbaz |
| G | 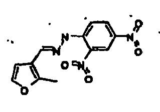 | N-(2,4-Dintro-phenyl)-N-(2-methyl -turan-3-ylmethylene)-hydrazine |
| H | 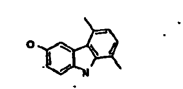 | 5,8-Dimethyl-8H-carbazo-3-ol |
| I | 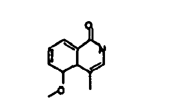 | 5-Methoxy-4-methyl-4a,5-dihydro-2H-isoquinoin-1-one |
| J | 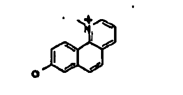 | 8-hydroxy-1-methyl-benzo[h]quinoinlum |
| K | 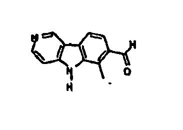 | 6-Methyl-5H-pyrido[4,3-b]indole-7-c arbaldehyde |

Le Tableau 1 représente le composé selon l'invention et le Tableau 2 les composés testés ayant une structure chimique différente des composés selon l'invention.

Le premier type de pré-messager correspond au Minx dérivé d'un transcrit d'adénovirus et dont l'épissage est constitutif (Zillmann, M. et al. (1988), Gel electrophoretic isolation of splicing complexes containing U1 small nuclear ribonucleoprotein particles. Mol.Cell Biol. 8, 814-821). Ce pré-messager est obtenu sous forme radioactive par transcription in vitro selon un protocole fourni par la société Promega en utilisant 1 µg de plasmide linéarisé, 20 unités de la polymérase SP6 et 5 µM [α-32P] UTP dans un volume de réaction de 25 µl.

50 finoles de ce transcrit sont utilisées pour des réactions d'épissage standard contenant dans 20 µl : 10 mM Triéthanolamine pH 7,9 ; 50 mM KCI, 0,1 mM EDTA ; 10% glycérol ; 0,5 mM DTT ; 20 mM créatine phosphate ; 2,5 mM ATP ; 2,5 mM MgCl₂ et 6% polyvinylalcool. On laisse incuber les réactions pendant 1h à 30°C.

Pour tester l'effet du composé selon l'invention, 1 µl de la dilution adéquate du composé est ajouté au début de la réaction sous forme d'une solution soluble dans du DMSO 10%.

Les ARNs produits au cours de la réaction d'épissage sont extraits, analysés sur un gel dénaturant de polyacrylamide 7% puis révélés par autoradiographie. Un exemple de l'inhibition de l'épissage du transcrit Minx obtenu avec 10 µM du composé C₂ (piste 4) est présenté sur la Figure 1.

Le second type de pré-messager M3S1 est dérivé du gène de la Béta-Globine humaine (Labourier, E. et al. (1999), Antagonism between RSF1 and SR proteins for both splice-site recognition in vitro and Drosophila development. Genes Dev. 13, 740-753) et son épissage est strictement dépendant d'une séquence auxiliaire ESE reconnue de manière spécifique par la protéine SR ASF/SF2. Les conditions de transcription, d'épissage et d'analyse des produits de ce pré-messager sont identiques à celles utilisées pour le pré-messager Minx.

Un exemple de l'inhibition de l'épissage de M3S1 obtenu avec 10 µM des composés C₂, C₃ et C₁₄ (pistes 4, 5 et 12) est présenté sur la Figure 2.

L'activité des produits a également été testée dans des réactions de formation de complexes d'épissage in vitro (Figure 3) comme décrit dans Pilch B. et al. (Specific inhibition of serine- and arginine-rich splicing factors phosphorylation, spliceosome assembly, and splicing by the antitumor drug NB-506. Cancer Res.2001. 61, 6876-6884).

Les réactions d'épissage du transcrit M3S1 en présence des différents composés selon l'invention réalisées dans les mêmes conditions que celles décrites pour la Figure 1 sont arrêtées après 30 minutes d'incubation par addition d'héparine et de glycérol à une concentration finale de 1 mg/ml et 15%, respectivement. Les complexes d'épissage sont séparés sur un gel d'acrylamide 5% non dénaturant et sont révélés par autoradiographie.

La Figure 3 montre un exemple d'inhibition de la formation des complexes d'épissage A et B au détriment de l'apparition de complexes abortifs pour les composés C₂, C₃ et C₁₄ (pistes 3, 4 et 9), utilisés à une concentration de 50 µM.

Le composé représenté dans le Tableau 1 est capable d'inhiber la formation des complexes d'épissage du transcrit M3S1 à une concentration comprise entre 10 µM et 50 µM.

### Exemple 2 : Inhibition in vivo de l'épissage ESE-dépendant de l'ARNm de la GFP (Green Fluorescent Protein)

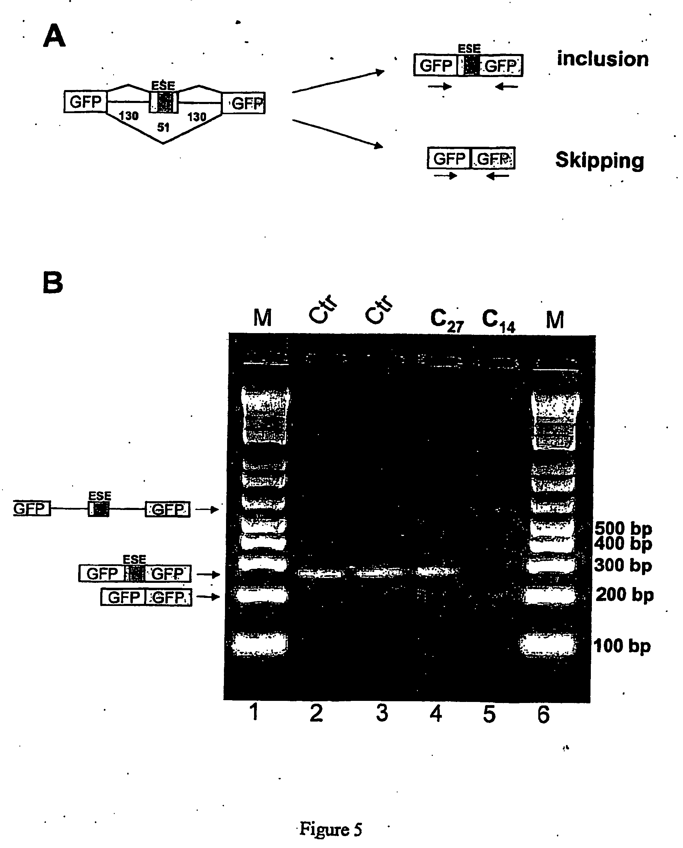

Afin de tester l'efficacité des dérivés d'indole *ex vivo*, des lignées cellulaires HeLa de fibroblastes ont été établies exprimant de façon stable un transgène correspondant à la GFP dont la séquence a été interrompue par une séquence ESE flanquée de deux introns identiques du gène de la Béta-Globine humaine décrit dans l'exemple 1 (voir Fig. 5A).

Pour détecter les ARN messagers issus de l'épissage de ce gène, la technique de RT-PCR a été utilisée avec des amorces dans la séquence GFP de part et d'autre de l'ESE et les produits de PCR ont été analysés sur gel d'agarose.

Dans presque toutes les lignées établies, un seul fragment de 250 paires de bases (pb) est amplifié par PCR (Fig. 5A, pistes 2 et 3) et il correspond à un ARN messager qui a inclus l'ESE entre les deux séquences-GFP.

Le résultat indique que l'ESE a un effet dominant et l'ARN messager produit après épissage contient les deux parties de la GFP interrompues par l'ESE (Fig. 5A, GFP-ESE-GFP).

A l'inverse, le traitement des cellules par des dérivés d'indole C₂₈ (piste 4) et C₁₄ (piste 5) fait apparaître un fragment de 194 pb, au détriment du fragment 250 pb, qui ne contient plus de séquence ESE entre les séquences GFP, démontrant ainsi que certains dérivés d'indole selon l'invention peuvent supprimer l'effet des ESE dans les cellules.

Certains composés représentés dans le Tableau 2 ont été testés à une concentration au moins égale à 1 µM et se sont avérés inefficaces dans ce test à cette concentration puisqu'ils n'ont pas induit un changement dans le profil d'épissage du transgène GFP-ESE.

Néanmoins, on peut signaler que l'ESE du transgène GFP-ESE utilisé dans les expériences décrites ci-dessus est spécifique de la protéine SR SF2/ASF et il est tout à fait probable que le composé selon l'invention représenté dans le Tableau 1 soit capable d'influencer l'épissage contrôlé par d'autres types d'ESE spécifiques des autres protéines SR (SC35, 9G8, SRp55, SRp40 ou SRp75).

**Tableau 3 :**

| | SF2 | SRp55 | SC35 |
|---|---|---|---|
| C1 | ++++ | ++++ | ++++ |
| C2 | ++++ | -- | ++++ |
| C3 | ++++ | ---- | ++++ |
| C4 | ---- | ---- | ---- |
| C5 | ++++ | ---- | ---- |
| C6 | | ---- | ---- |
| C7 | ++++ | ---- | ---- |
| C8 | ++++ | ---- | ++++ |
| C9 | ++++ | ---- | ---- |
| C10 | ---- | ---- | ---- |
| C11 | +/- | ---- | ++++ |
| C12 | ++++ | ++++ | ++++ |
| C13 | ++++ | / | ---- |
| C14 | ++++ | ++++ | ---- |
| C15 | | ---- | ++++ |
| C16 | +/- | ++++ | ++++ |
| C17 | ---- | ---- | ++++ |
| C18 | ---- | ---- | ++++ |
| C19 | +/- | ++++ | ++++ |
| C20 | ---- | ---- | ---- |
| C21 | ++++ | ---- | ++++ |
| C22 | ---- | ---- | ---- |
| C23 | ---- | ---- | ---- |
| C24 | ---- | ---- | ---- |
| C25 | ---- | ---- | ++++ |
| C26 | ++++ | ---- | ++++ |
| C27 | ---- | ---- | ++++ |
| C28 | +/- | ---- | / |
| C29 | ++++ | ---- | / |
| C30 | ++++ | ++++ | / |
| C31 | ++++ | ++++ | / |
| C32 | ++++ | ++++ | / |
| C33 | ++++ | ++++ | / |
| C34 | ++++ | ---- | / |
| C35 | ++++ | ---- | / |
| C36 | ++++ | ++++ | / |
| C37 | ++++ | ++++ | / |
| C38 | ++++ | ++++ | / |
| C39 | ++++ | ++++ | / |
| C40 | ++++ | ++++ | / |
| C41 | ++++ | ++++ | / |
| C42 | ---- | +++ | / |
| C43 | ++++ | ++++ | / |
| C44 | ++++ | ---- | / |
| C45 | ++++ | ---- | / |
| C46 | ++++ | ++++ | / |
| C47 | ++++ | ---- | / |
| C48 | ++++ | ++++ | / |
| C49 | ++++ | ++++ | / |
| C50 | ---- | ++++ | / |
| C51 | ++++ | ---- | / |
| C52 | ++++ | ++++ | / |
| C53 | ++++ | ++++ | / |
| C54 | ++++ | +++ | / |
| C55 | +/- | +++ | / |
| C56 | ++++ | +++ | / |
| C57 | ---- | ++++ | / |
| C58 | ++++ | ---- | / |
| C59 | +++++ | ++++ | / |
| C60 | +++++ | ++++ | / |
| C61 | +++++ | ++++ | / |
| C62 | +++++ | ++++ | / |
| C63 | ++++ | ++++ | ++++ |
| C64 | ++++ | ++++ | / |
| C65 | ++++ | ++++ | / |
| C66 | ++++ | ++++ | / |
| C67 | ++++ | ++++ | / |
| C68 | +/- | ---- | / |
| C69 | ++++ | ++ | / |
| C70 | +/- | ---- | / |
| C71 | +/- | ---- | / |
| C72 | +/- | ---- | / |
| C73 | ++++ | ---- | / |
| C74 | ++++ | ++ | / |
| C75 | ++++ | +++ | / |
| C76 | +/- | +++ | / |
| C77 | ---- | + | / |
| C78 | ---- | + | / |
| C79 | ---- | ++ | / |
| C80 | +/- | ++ | / |
| C81 | ++++ | +++ | / |
| C82 | | ++ | / |
| C83 | ++++ | ---- | / |
| C84 | ++++ | ++++ | / |
| C85 | +/- | + | / |
| C86 | ++++ | ++ | / |
| C87 | +/- | ++ | / |
| C88 | +/- | ++ | / |
| C89 | ++++ | +++ | / |
| C90 | ++++ | --- | / |
| C91 | ++++ | --- | / |
| C92 | ++++ | --- | / |
| C93 | +/- | ++++ | / |
| C94 | ++++ | ++++ | / |
| C95 | +/- | ++++ | / |
| C96 | ++++ | ++++ | / |
| C97 | ++++ | --- | / |
| C98 | ++++ | ++ | / |
| C99 | ++++ | ---- | / |
| C100 | ++++ | ---- | / |

### Exemple 3 : Inhibition in vivo de l'épissage ESE-dépendant de l'ARNm de la sous-unité E1α de la pyruvate déshydrogénase (Exemple de référence)

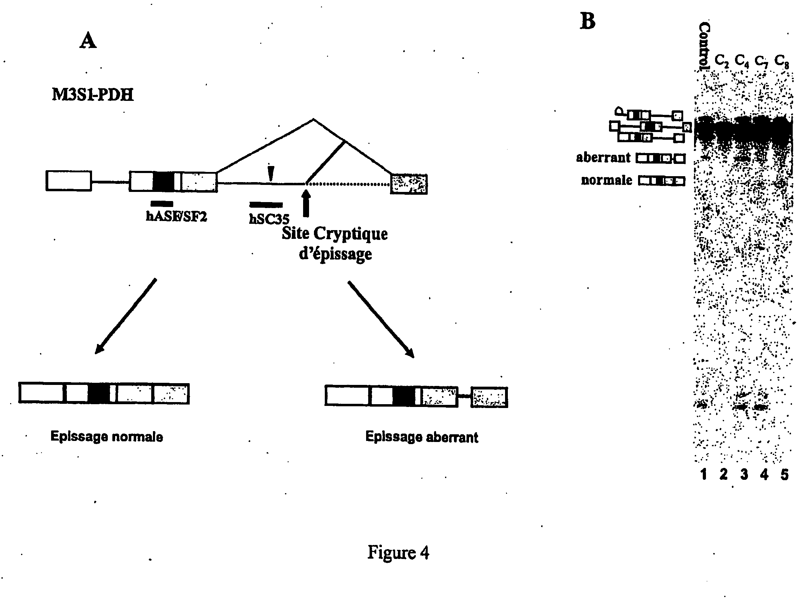

Afin de démontrer la sélectivité de l'action des composés selon l'invention sur les séquences ESE, il a été décidé d'utiliser un autre substrat modèle comportant deux introns et dont l'épissage dépend de deux séquences ESEs différentes. Dans ce substrat les séquences correspondant à l'exon 7-intron 7-exon 8 du gène codant pour la sous unité E1α de la puryvate désydrogènase (PDH E1α) sont insérées en aval de la séquence de M3S1 (M3S1-PDH, voir Figure 4A). L'intron 7 de ce transcrit contient une mutation ponctuelle qui crée un site de haute affinité pour la fixation de la protéine SR hSC35. Cette mutation qui est à l'origine de l'extinction de l'expression de PDH E1α chez un patient atteint du syndrome de Leigh (une encéphalopathie de l'enfant) entraîne l'apparition d'un site cryptique 46 nucléotides en aval du site 5' d'épissage authentique (Fig. 4A). Ce substrat, susceptible de donner lieu à deux produits, incluant ou pas les 46 nucléotides de l'intron 7 de la PDH, est idéal pour déterminer la spécificité des composés vis-à-vis de séquences ESE différentes présentes au sein du même transcrit. Le composé C₂ qui inhibe M3S1 abolit complètement l'épissage de M3S1-PDH (Fig. 4B, compare pistes 1 et 2). Une inhibition de M3S1-PDH est également observée avec le composé C₈ (Fig. 4B, piste 5), indiquant que la séquence ESE contenue dans M3S1 est requise pour déclencher le premier évènement d'épissage servant à éliminer l'intron 1. A l'inverse, le composé C₄, inactif sur M3S1, n'a aucun effet (Fig. 4B piste 3). Cependant, le crible d'autres composés n'altérant pas l'épissage de M3S1, a révélé de façon surprenante que le composé C₇ bloque la formation des espèces d'ARNs issues de l'épissage PDH incluant les 46 nucléotides de l'intron 7 mais n'a aucun effet sur celles dérivées d'un épissage normale PDH (Fig. 4B, piste 4). Le composé C₇ est donc un excellent inhibiteur de l'épissage aberrant dépendant de l'ESE hSC35 mais pas de l'épissage authentique. Ce composé peut donc être envisagé pour traiter le malade atteint de cette encéphalopathie.

### Exemple 4 : Inhibition de la multiplication du VIH par les composés de l'invention (Exemple de référence)

Le virus du SIDA, comme pratiquement tous les rétrovirus, a recours à l'épissage alternatif pour exprimer des gènes essentiels à sa réplication. En effet, la version du virus qui s'intègre dans le génome des cellules humaines est transcrite sous forme d'un précurseur unique qui, par épissage alternatif, génère 40 ARN messagers différents codant pour des protéines virales essentielles à sa réplication (Furtado et al., 1991. Analysis of alternatively spliced human immunodeficiency virus type-1 mRNA species, one of which encodes a novel tat-env fusion protein.Virology, 185 : 258-270 ; Purcell and Martin, 1993. Alternative splicing of human immunodeficiency virus type 1 mRNA modulates viral protein expression, replication, and infectivity. J. Virol., 67 : 6365-78). Ces évènements d'épissage sont contrôlés par plusieurs séquences régulatrices de type ESE dont certaines sont localisées en aval de sites d'épissage responsables de l'expression des protéines clefs de la réplication virale comme tat, rev, vpu, env et nef (Caputi et al., 2004. A bidirectional SF2/ASF- and SRp40-dependent splicing enhancer regulates human immunodeficiency virus type 1 rev, env, vpu, and nef gene expression. J. Virol. 78 : 6517-26 ; Pongoski et al., 2002. Positive and negative modulation of human immunodeficiency virus type 1 Rev function by cis and trans regulators of viral RNA splicing. J. Virol. 76 : 5108-20).

Puisque des composés inhibent l'utilisation de sites d'épissage dépendants de séquences ESE, il a été testé leur efficacité à bloquer la réplication virale. Pour cela, nous avons utilisé la lignée lymphocytaire humaine U1, chroniquement infectée par le VIH (Folks et al., 1988. Characterization of a promonocyte clone chronically infected with HIV and inducible by 13-phorbol-12-myristate acetate. J. Immunol., 140: 1117-1122), et qui produit des quantités importantes de virus après stimulation par le PMA (Phorbol Myristate Acetate). Cette lignée cellulaire constitue, de ce fait, un excellent modèle pour mimer la transition entre la phase de latence et la phase de production virale observée chez des patients infectés par le VIH.

Les résultats de cette expérience sont présentés en Figure 6 où les cellules U1 (5 x 10⁵) ont été traitées par 50 nM de PMA en absence (Fig. 6, piste 1) ou en présence de 2,5 µM des composés C₄₇, C₉₇, C₅₈, C₅₇, C₉₂, C₉₁, C₉₀, C₈₃, C₇₃, C₃₄, C₅₁, C₄₅, C₁₃, C₁₀, C₄₄, C₆, C₇, C₄₁, C₅₀, C₃₂, C₂, C₁, C₂₉, C₃₅ et C₉₉ (Fig. 6, pistes 3-27, respectivement). Après 24h de traitement, les transcrits du virus ont été amplifiés par RT-PCR en utilisant des amorces spécifiques du VIH, BSS et SJ4.7A (Jacquenet et al., 2001, A second exon splicing silencer within human immunodeficiency virus type 1 tat exon 2 represses splicing of Tat mRNA and binds protein hnRNP H. J. Biol. Chem. 276 : 40464-75) et un traceur radioactif (α-32P) CTP. Les produits d'amplification sont analysés sur gel dénaturant de polyacrylamide 7% puis révélés par autoradiographie. Parmi les 29 composés testés, les composés C₄₇, C₉₇, C₅₇, C₉₂, C₉₁, C₈₃, C₅₁, C₁₃, C₁₀, C₄₄, C₄₁, C₅₀, C₃₂, C₂, C₁, C₂₉, C₃₅ et C₉₉ (pistes 3, 4, 6-8, 10, 13, 15-17, 20-27, respectivement) se sont révélés d'excellents inhibiteurs de la multiplication du VIH car aucune amplification du virus n'a été détectée dans les cellules traitées par ces composés.

### Exemple 5 : la 10-Chloro-2,6-diméthyl-2Hpyrido[3',4':4,5]pyrrolo [2,3-g]isoquinoline

A la solution refroidie à -10 °C de 10-chloro-6-méthyl-5H-pyrido[3',4':4,5]pyrrolo[2,3-g]isoquinoline (400 mg; produit connu, déjà publié) dans le N,N-diméthylacétamide (20 mL) est ajouté NaH à 50% (90 mg). Le mélange est agité pendant 30 min. puis CH₃I (0,11 mL) est introduit et le mélange est agité toujours à -10 °C pendant 2,5 h. De l'eau et CH₂Cl₂ sont ajoutés et la phase organique est séparée, séchée (MgSO₄) et évaporée à sec. Le résidu obtenu est chromatographié sur colonne de silice en éluant successivement par CH₂Cl₂-EtOH (9-1: v/v) puis par EtOH-NEt₃ (95-5: v/v) pour donner respectivement la 10-chloro-5,6-diméthyl-5H-pyrido[3',4':4,5]pyrrolo[2,3-g]isoquinoline (66 mg, 16% de rendement) et la 10-chloro-2,6-diméthyl-2H-pyrido[3',4':4,5]pyrrolo[2,3-g]isoquinoline attendue (138 mg, 32 % de rendement) sous forme des microcristaux jaunes (point de fusion > 260°C). RMN en accord.

### Exemple 6 : la (9-Méthoxy-5-méthyl-6H-pyrido[4,3-b]cabazol-1-yl)-(2,2,6,6-tétraméthyl-pipéridin-4-yl)-amine (Exemple de référence)

Le mélange de 1-chloro-9-méthoxy-5-méthyl-6H-pyrido[4,3-b]carbazole (1,0 g; produit connu, déjà publié) et de 4-amino-2,2,6,6-tétraméthylpiperidine (1,0 g) est chauffé à 150°C pendant 21 h. L'excès de l'amine est éliminé par évaporation sous pression réduite. De l'eau est ajoutée au résidu obtenu pour former un solide. Ce dernier est filtré, lavé à l'eau, puis recristallisé du xylène pour donner la 9-méthoxy-5-méthyl-6H-pyrido[4,3-b]carbazol-1-yl)-(2,2,6,6-tétraméthyl-pipéridin-4-yl)-amine attendue (1,0 g, 71 % de rendement) sous forme des microcristaux jaunes (point de fusion: 223-5°C). RMN en accord.

### Exemple 7 : l'acide N-éthyl-N-[3-9-méthoxy-5,11-diméthyl-6H-pyrido[4,3-b]carbazol-1-ylamino)-propyl]-succinimique. (Exemple de référence)

Une solution de 100 mg d'anhydride succinique (1 mM) et de 376 mg (1 mM) de N-éthyl-N'-(9-méthoxy-5,11-diméthyl-6H-pyrido[4,3-b]carbazol-1-yl)-propane-1,3-diamine dans 100 ml de toluène sec est portée à reflux durant 5 h. Le précipité obtenu après refroidissement est essoré et recristallisé de l'acétone. On obtient 270 mg (54,6 %) du produit désiré sous forme de solide F ∼150°. Analyse centésimale pour C₂₇H₃₂N₄O₄ + H₂O ; Calc. : C, 65,57 ; H, 6,93 ; N, 11.33 ; Exp. : C, 65,18 ; H, 6,88 ; N, 10,95. RMN et SM en accord.

### Exemple 8: l'acide N-éthyl-N-[3-(6-méthyl-5H-pyrido[3',4' : 4,5]pyrrolo [2,3-g]isoquinolin-10-ylamino)-propyl]-succinamique (Exemple de référence)

Une solution de 75 mg d'anhydride succinique (0.75 mM) et de 250 mg (0 .75 mM) de N-éthyl-N'-(6-méthyl-5H-pyrido[3',4' : 4,5] pyrrolo[2,3-g] isoquinoléine-10-yl)propane-1,3-diamine dans 80 ml de toluène sec est portée à reflux durant 4 h. Le précipité obtenu après refroidissement est essoré et recristallisé de l'acétone. On obtient 240 mg (65,6 %) sous forme de solide F ∼150°. Analyse centésimale pour C₂₄H₂₇N₅O₃ + 3H₂O ; Calc. : C, 59,12 ; H, 6,82 ; N, 14,37 ; Exp. : C, 59,14 ; H, 6,88 ; N, 14,17. RMN et SM en accord.

### Exemple 9 : le 9-méthoxy-5-méthyl-4,6-dihydro-3H-pyrido[4,3-b]carbazole (Exemple de référence)

Une suspension de 1,5 g de 2-(-6-méthoxy-1-méthyl-9H-carbazel-2yl)-éthylamine dans 15 ml de formiate d'éthyle est portée à reflux durant 20 h. La solution obtenue est concentrée sous vide et la pâte obtenue est reprise dans le dichlorométhane. Après évaporation complète sous pression réduite le solide obtenu est broyé puis lavé au pentane pour fournir 1,6 g (98 %) du dérivé intermédiaire N-formylé.

Les 1,6 g de cet intermédiaire sont dissous dans 120 ml de toluène sec et l'ensemble est porté à reflux dans un tricol équipé d'un Dean Stark. 12 ml de POCl₃ sont additionnés goutte à goutte en 10 min. et le reflux est maintenu durant 24 h. Après refroidissement et évaporation du toluène et du POCl₃, sous pression réduite, le solide est repris dans 500 ml d'HCL 2N à ébullition. Un léger insoluble est filtré et, de la solution refroidie, on essore le chlorhydrate. Celui-ci est repris dans NH₄OH 2N (jusqu'à pH 12). Le précipité jaune essoré après séchage fournit 1,4 g (92 %) du produit désiré. RMN en accord.

### Exemple 10 : 1a N-(3-amino-propyl)N'-[3-(3-méthoxy-8-méthyl-7H-benzo[e]pyrido[4,3-b]indol-11-ylamino)-propyl]butane-1,4-diamine (Exemple de référence)

Le mélange de 11-chloro-8-méthyl-3-méthoxy-7H-benzo[e]pyrido[4,3-b]indole (210 mg; produit connu, déjà publié) et de spermine (1,0 g) est chauffé à 170°C pendant 18 h. L'excès de l'amine est éliminé par évaporation sous pression réduite et le résidu obtenu est repris dans CH₂Cl₂ puis lavé à l'eau. La phase organique est séchée (MgSO₄) et évaporée à sec..La base libre obtenue est ensuite dissoute dans l'éthanol absolu bouillant (15 mL) puis verséé dans une solution contenant l'acide maléique (410 mg) et éthanol (10 mL). Le précipité obtenu est filtré à 20°C, lavé à l'éthanol et séché à l'abri de l'humidité pour donner 270 mg (39% de rendement) de sel tétramaléate du produit attendu. Analyse centésimale pour C₄₃H₅₄N₆O₁₇ + 2H₂O; Calc. : C, 53,64 ; H, 6,03 ; N, 8.73 ; Exp. : C, 53,83 ; H, 6,01 ; N, 8,79. RMN en accord.

### Exemple 11 : la N-(3-Méthoxy-8-méthyl-7H-benzo[e]pyrido[4,3-b]indol-11-yl)-hexane-1,6-diamine (Exemple de référence)

Le mélange de 11-chloro-8-méthyl-3-méthoxy-7H-benzo[e]pyrido[4,3-b]indole (360 mg; produit connu, déjà publié) et de l'hexane-1,6-diamine (5 mL) est chauffé à 170°C pendant 18 h. L'excès de l'amine est éliminé par évaporation sous pression réduite et le résidu obtenu est repris dans CH₂Cl₂ puis lavé à l'eau. La phase organique est séchée (MgSO₄) et évaporée à sec. La base libre obtenue est ensuite dissoute dans l'éthanol absolu bouillant (15 mL) puis verséé dans une solution contenant l'acide maléique (648 mg) et éthanol (10 mL). Le précipité obtenu est filtré à 20°C, lavé à l'éthanol et séché à l'abri de l'humidité pour donner 700 mg (89% de rendement) de sel bimaléate du produit attendu. Analyse centésimale pour C₃₁H₃₆N₄O₉ + 2H₂O; Calc. : C, 57,76 ; H, 6,21 ; N, 8.69 ; Exp. : C, 58,22 ; H, 9,91 ; N, 8,47. RMN en accord.

### Exemple 12 : 1a N-[3-(3-méthoxy-10-méthyl-11H-benzo[g]pyrido[4,3-b]indol-7-ylamino)-propyl]-propane-1,3-diamine (Exemple de référence)

Le mélange de 7-chloro-10-méthyl-3-méthoxy-11H-benzo[g]pyrido[4,3-b]indole (335 mg; produit connu, déjà publié) et de la N-(3-aminopropyl)-1,3-propanediamine (8 mL) est chauffé à 170°C pendant 18 h. L'excès de l'amine est éliminé par évaporation sous pression réduite et le résidu obtenu est repris dans CH₂Cl₂ puis lavé à l'eau. La phase organique est séchée (MgSO₄) et évaporée à sec. La base libre obtenue est ensuite dissoute dans l'éthanol absolu bouillant (15 mL) puis verséé dans une solution contenant l'acide maléique (620 mg) et éthanol (10 mL). Le précipité obtenu est filtré à 20°C, lavé à l'éthanol et séché à l'abri de l'humidité pour donner 460 mg (53% de rendement) de sel trimaléate du produit attendu. Analyse centésimale pour C₃₅H₄₁N₅O₁₃ + H₂O; Calc. : C, 55,48 ; H, 5,68 ; N, 9.25 ; Exp. : C, 55,01 ; H, 5,68 ; N, 9,14. RMN en accord

### Exemple 13: la N-[3-(3-méthoxy-8-méthyl-7H-benzo[e]pyrido[4,3-b]indol-11-ylamino)-propyl]-propane-1,3-diamine (Exemple de référence)

Le mélange de 11-chloro-8-méthyl-3-méthoxy-7H-benzo[e]pyrido[4,3-b]indole (290 mg; produit connu, déjà publié) et de la N-(3-aminopropyl)-1,3-propanediamine (5 mL) est chauffé à 170°C pendant 30 h. L'excès de l'amine est éliminé par évaporation sous pression réduite et le résidu obtenu est repris dans CH₂Cl₂ puis lavé à l'eau. La phase organique est séchée (MgSO₄) et évaporée à sec. La base libre obtenue est ensuite dissoute dans l'éthanol absolu bouillant (15 mL) puis verséé dans une solution contenant l'acide maléique (620 mg) et éthanol (10 mL). Le précipité obtenu est filtré à 20°C, lavé à l'éthanol et séché à l'abri de l'humidité pour donner 360 mg (48% de rendement) de sel trimaléate du produit attendu. Analyse centésimale pour C₃₅H₄₁N₅O₁₃ + 1,5 H₂O; Calc. : C, 54,83 ; H, 5,74 ; N, 9.14 ; Exp. : C, 54,98 ; H, 5,91 ; N, 8,76. RMN en accord.

## Revendications

1. Dérivé d'indole correspondant à la formule I suivante : 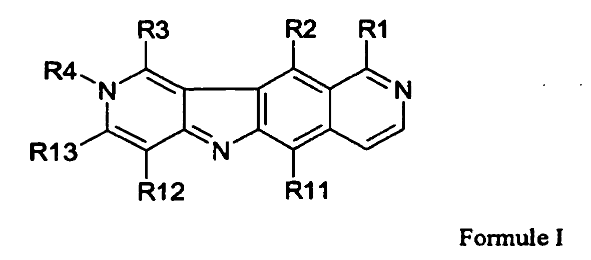 dans laquelle :
R1 représente :
• un atome d'halogène ou un groupement -C=N-OH ou -O-C(=O)(CH₃) ou -C≡ N, ou
• un groupement -N-R6R7,
où R6 et R7 représentent indépendamment l'un de l'autre :
• un atome d'hydrogène,
• un cycle en C6, saturé ou insaturé, comportant éventuellement un atome d'azote, et éventuellement substitué par un ou plusieurs groupements alkyles en C1 à C3, ou
• un groupement alkyle de C1 à C13 linéaire ou ramifié et/ou insaturé, dans lequel un ou plusieurs atomes de carbone peut être substitué par un atome d'azote, ledit groupement alkyle étant éventuellement substitué par un ou plusieurs groupements -OH et/ou = O et/ou par un groupement tel que : 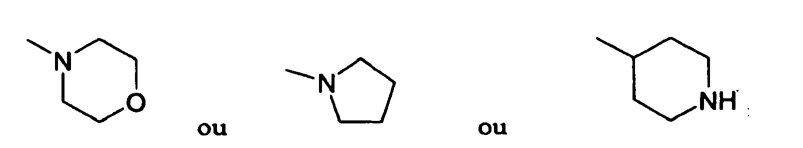
ou 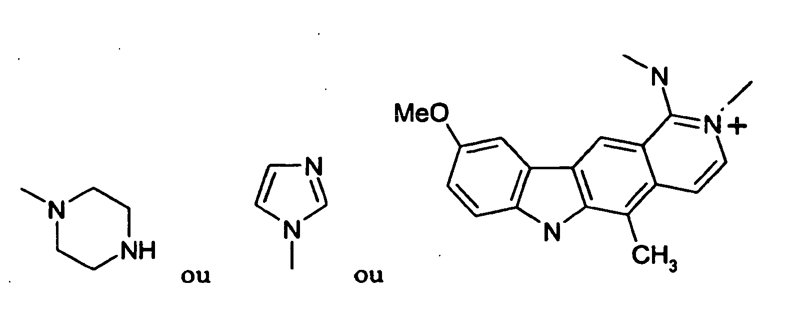 ledit groupement étant éventuellement substitué par un groupement alkyle en C1 à C3 lui-même éventuellement substitué par un groupement amine,
• un groupement -NH-R8
où R8 représente un groupement alkyle-N-R9R10
où le groupement alkyle représente un groupement de C1 à C13 linéaire ou ramifié, éventuellement insaturé et/ou substitué par un ou plusieurs groupements alkyle en C1 à C3 et/ou groupements hydroxyle,
R9 et R10 représentent chacun indépendamment l'un de l'autre un atome d'hydrogène ou un groupement alkyle en C1 à C4 éventuellement substitué par un ou plusieurs groupements hydroxyle ét/ou oxo,
R2 représente un atome d'hydrogène, un groupement méthyle ou un groupement - NH-(CH₂)₃-N(CH₃)₂,
R3 représente un atome d'hydrogène, un atome d'halogène ou un groupement méthyle, amine ou méthoxyméthyle ou -NH-R8 tel que défini précédemment,
R4 représente un groupement alkyle en C1-C6, un groupement hydroxyle ou un groupement méthoxy éventuellement substitué par un groupement phényle,
R11 et R12 représentent indépendamment l'un de l'autre un atome d'hydrogène ou un groupement alkyle en C1-C3,
R13 représente un atome d'hydrogène ou un groupement méthyle, et
les sels pharmaceutiquement acceptables desdits composés et/ou mélanges de ceux-ci.

2. Dérivés d'indole selon la revendication 1 de formule I, dans laquelle :
R1 représente un atome d'halogène, un groupement amine, -N-R₆R₇ ou -NH-R8,
R2 représente un atome d'hydrogène ou un groupement méthyle,
R3 représente un atome d'hydrogène ou un groupement NH-R8,
R4 représente un groupement méthyle,
R11 représente un atome d'hydrogène ou un groupement méthyle, et
R6, R7, R8, R9, R10, R12 et R13 sont tels que définis dans la revendication 1.

3. Dérivés selon la revendication 1, dans lesquels le composé I est le composé 10-chloro-2,6-diméthyl-2H-pyrido[3',4':4,5]pyrrolo[2,3-g]isoquinoline :

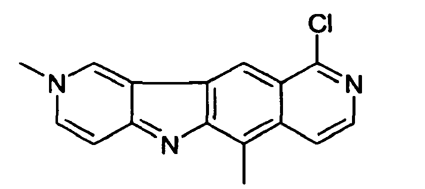

4. Dérivés selon l'une quelconque des revendications 1 à 3 pour leur utilisation comme médicament.

5. Dérivés selon la revendication 4, **caractérisés en ce que** le médicament est destiné à traiter les maladies liées au processus d'épissage des ARN pré-messagers dans la cellule,
(a) notamment les maladies génétiques résultant de l'altération des processus d'épissage, notamment le syndrome de Frasier, la démence fronto-temporaire liée au chromosome 17 (une forme du Parkinson), le syndrome de Leigh (type d'encéphalopathie), la mucoviscidose atypique, les neuropathologies dont notamment l'Alzheimer lié a une mutation de la protéine Tau, l'amyotrophie qui touche le gène SMN (Survival of Motor Neuron), la dépression liée a un dérèglement de l'épissage de la sérotonine,
(b) les maladies d'origine virale et pour lesquelles des séquences ESE sont identifiées pour l'épissage, telles que le SIDA. »

6. Dérivés selon la revendication 4 ou 5, **caractérisés en ce que** ledit médicament comprend également un excipient permettant de formuler les composés selon la formule I.

7. Dérivés selon l'une des revendications 4 à 6, **caractérisés en ce que** ledit médicament se présente sous forme solide ou liquide pour être préparé et administré par voie intraveineuse.

## Claims

1. An indole derivative corresponding to following formula I: 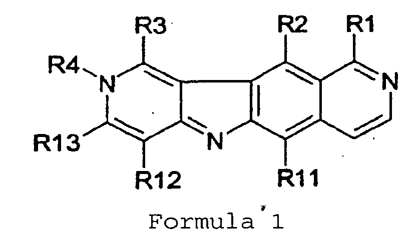 wherein:
R1 represents:
• a halogen atom or a -C=N-OH or -O-C (=O) (CH₃) or -C≡N group, or
• an -N-R6R7 group,
wherein R6 and R7 represent independently of one another:
• a hydrogen atom,
• a ring at C6, saturated or unsaturated, optionally containing an atom of nitrogen, and optionally substituted by one or more alkyl groups at Cl to C3, or
• a linear, branched and/or unsaturated alkyl group at C1 to C13 wherein one or more atoms of carbon can be substituted by an atom of nitrogen, the aforementioned alkyl group being optionally substituted by one or more -OH and/or =O groups and/or by a group such as: 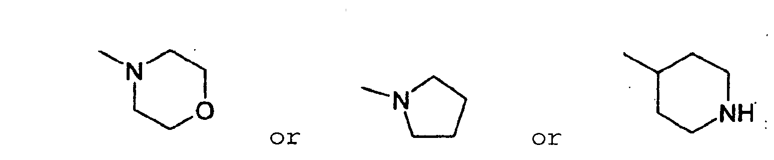 or 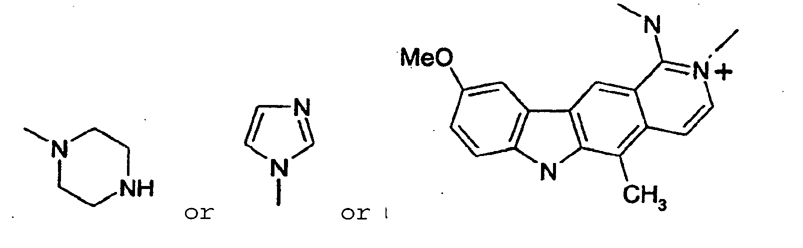
the aforementioned group being optionally substituted by an alkyl group at C1 to C3 which itself is optionally substituted by an amino group,
- an -NH-R8 group
wherein R8 represents an alkyl-N-R9R10 group
wherein the alkyl group represents a linear or branched group at C1 to C13 optionally unsaturated and/or substituted by one or more alkyl groups at C1 to C3 and/or hydroxyl groups,
R9 and R10 represent independently of one another a hydrogen atom or an alkyl group at C1 to C4 optionally substituted by one or more hydroxyl and/or oxo groups,
R2 represents a hydrogen atom, a methyl group or a -NH-(CH₂)₃-N(CH₃)₂ group,
R3 represents a hydrogen atom, a halogen atom or a methyl, amino or methoxymethyl group, or -NH-R8 such as defined previously,
R4 represents an alkyl group at C1-C6, a hydroxyl group or a methoxy group optionally substituted by a phenyl group,
R11 and R12 represent independently of one another a hydrogen atom or an alkyl group at C1-C3,
R13 represents a hydrogen atom or a methyl group,
and the pharmaceutically acceptable salts of the aforementioned compounds, and/or mixtures of same.

2. Indole derivatives according to claim 1 -of formula I, wherein:
R1 represents an atom of halogen, an amino, -N-R₆R₇ or -NH-R8 group,
R2 represents a hydrogen atom or a methyl group,
R3 represents a hydrogen atom or an NH-R8 group,
R4 represents a methyl group,
R11 represents a hydrogen atom or a methyl group, and
R6, R7, R8, R9, R10, R12 and R13 are such as defined in claim 1.

3. Derivatives according to claim 1, wherein compound I is the compound 10-chloro-2,6-dimethyl-2H-pyrido[3',4':4,5]pyrrolo[2,3-g]isoquinoline:

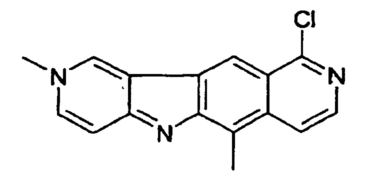

4. Derivatives according to any of claims 1 to 3 for their use as a drug.

5. Derivatives according to claim 4,
**characterized in that** the drug is intended to treat diseases related to pre-messenger RNA splicing process within the cell,
(a) genetic diseases resulting from splicing-process modification, notably Frasier syndrome, frontotemporal dementia related to chromosome 17 (a form of Parkinson's), Leigh syndrome (a type of encephalopathy), atypical cystic fibrosis, certain neuropathologies, such as in particular the Alzheimer, related to a Tau protein mutation, amyotrophy that influences the SMN (survival motor neuron) gene, depression related to disturbances in serotonin splicing,
(b) diseases of viral origin for which ESE splicing sequences are identified, such as AIDS.

6. Derivatives according to claim 4 or 5,
**characterized in that** the aforementioned drug also includes an excipient enabling the formulation of compounds according to formula I.

7. Derivatives according to one of claims 4 to 6, **characterized in that** the aforementioned drug is provided in solid or liquid form to be prepared and administered by intravenous route.

## Patentansprüche

1. Indolderivat, das der folgenden Formel I entspricht: 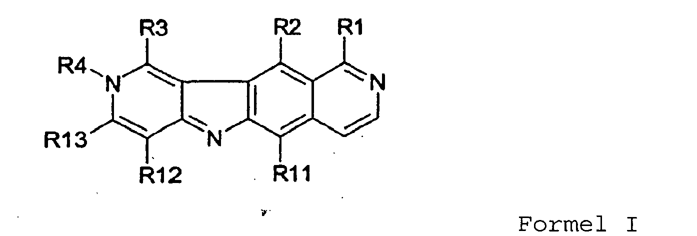 in welcher:
R₁ darstellt:
• ein Halogenatom oder eine -C=N-OH- oder -O-C(=O)(CH₃)- oder -C≡N-Gruppe, oder
• eine -N-R6R7-Gruppe,
wobei R6 und R7 unabhängig voneinander darstellen:
• ein Wasserstoffatom,
• einen C6-Ring, gesättigt oder ungesättigt, der eventuell ein Stickstoffatom umfasst, und eventuell von einem oder mehreren C₁-C₃-Alkylgruppen ersetzt wird,
• eine lineare oder verzweigte und/oder ungesättigte C₁-C₃-Alkylgruppe, in der ein oder mehrere Kohlenstoffatome von einem Stickstoffatom ersetzbar sind, wobei die Alkylgruppe eventuell ersetzt wird von einer oder mehreren -OH- und/oder =O-Gruppen und/oder von einer Gruppe wie 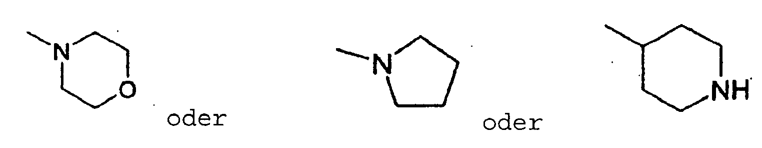 oder
oder 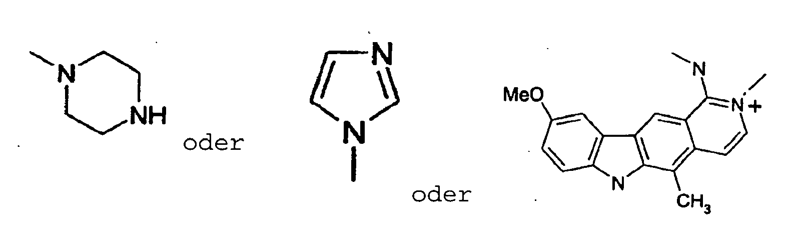 oder
wobei die Gruppe eventuell von einer C₁-C₃-Alkylgruppe ersetzt wird, die selbst eventuell von einer Amingruppe ersetzt wird,
• eine -NH-R8-Gruppe,
wobei R8 eine Alkyl-N-R9R10-Gruppe darstellt,
wobei die Alkylgruppe eine lineare oder verzweigte C₁-C₁₃-Gruppe darstellt, eventuell ungesättigt und/oder ersetzt von einer oder mehreren C₁-C₃-Alkylgruppen und/oder Hydroxylgruppen,
wobei R9 und R₁₀ jeweils unabhängig voneinander ein Wasserstoffatom darstellen oder eine C₁-C₄-Alkylgruppe, die eventuell von einer oder mehreren Hydroxyl- und/oder Oxogruppen ersetzt wird,
R2 ein Wasserstoffatom, eine Methylgruppe oder eine NH-(CH₂)₃-N(CH₃)₂-Gruppe darstellt,
R3 ein Wasserstoffatom, ein Halogenatom oder eine Methyl-, Amin- oder Methoxymethyl- oder -NH-R8-Gruppe darstellt, wie zuvor definiert,
R4 eine C₁-C₆-Alkylgruppe, eine Hydroxylgruppe oder eine Methoxygruppe darstellt, die eventuell von einer Phenylgruppe ersetzt wird,
R11 und R12 unabhängig voneinander ein Wasserstoffatom oder eine C₁-C₃-Alkylgruppe darstellen,
R13 ein Wasserstoffatom oder eine Methylgruppe darstellt und
die pharmazeutisch akzeptablen Salze der Verbindungen und/oder Gemische derselben.

2. Indolderivate nach Anspruch 1 der Formel I, in welcher:
R1 ein Halogenatom, eine Amin-, -N-R₆R₇- oder -NH-R8-Gruppe darstellt,
R2 ein Wasserstoffatom oder eine Methylgruppe darstellt,
R3 ein Wasserstoffatom oder eine NH-R8-Gruppe darstellt,
R4 eine Methylgruppe darstellt,
R11 ein Wasserstoffatom oder eine Methylgruppe darstellt und
R6, R7, R8, R9, R10, R12 und R13 derart sind, wie in Anspruch 1 definiert.

3. Derivate nach Anspruch 1, in welchen die Verbindung I die Verbindung 10-Chloro-2,6-dimethyl-2H-pyrido[3',4':4,5]pyrrolo[2,3-g]isochinolin ist:

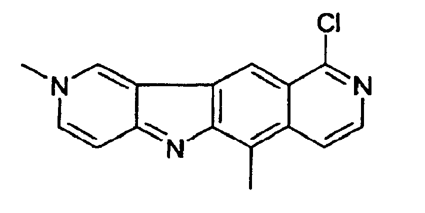

4. Derivate nach einem der Ansprüche 1 bis 3 für ihre Verwendung als Arzneimittel.

5. Derivate nach Anspruch 4, **dadurch gekennzeichnet, dass** das Arzneimittel dazu bestimmt ist, Erkrankungen zu behandeln, die mit dem Prozess des Splicings der prä-mRNA in der Zelle verbunden sind,
(a) vor allem die genetischen Erkrankungen, die aus der Alteration der Splicingprozesse resultieren, vor allem das Frasier-Syndrom, die mit dem Chromosom 17 verbundene frontotemporäre Demenz (eine Form von Parkinson), das Leigh-Syndrom (Art der Enzephalopathie), die atypische Mukoviszidose, die Neuropathologien wie vor allem den mit einer Mutation des Tau-Proteins verbundene Alzheimer, die das Gen SMN (Survival of Motor Neuron) betreffende Amyotrophie, die mit einer Deregulierung des Serotoninsplicings verbundene Depression,
(b) Viruserkrankungen und Erkrankungen, für die ESE-Sequenzen für das Splicing identifiziert sind, wie zum Beispiel AIDS.

6. Derivate nach Anspruch 4 oder 5, **dadurch gekennzeichnet, dass** das Arzneimittel weiterhin einen Trägerstoff umfasst, der es erlaubt, Verbindungen nach Formel I zu formulieren.

7. Derivate nach Anspruch 4 bis 6, **dadurch gekennzeichnet, dass** sich das Arzneimittel in fester oder flüssiger Form darstellt, um präpariert und auf intravenösem Weg verabreicht zu werden.
